(19) Europäisches Patentamt
European Patent Office
Office européen des brevets

(11) **EP 2 581 731 B1**

(12) **EUROPÄISCHE PATENTSCHRIFT**

(45) Veröffentlichungstag und Bekanntmachung des Hinweises auf die Patenterteilung:
**03.01.2018 Patentblatt 2018/01**

(51) Int Cl.:
*G01N 21/88* (2006.01)　　*G01N 21/94* (2006.01)
*A61B 90/90* (2016.01)

(21) Anmeldenummer: **12188355.7**

(22) Anmeldetag: **12.10.2012**

(54) **Vorrichtung und Verfahren zum Erkennen von Anomalien an Instrumenten**

Apparatus and method for detecting anomalies of instruments

Dispositif et procédé de détection d'anomalies sur des instruments

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2011 DE 102011054448**

(43) Veröffentlichungstag der Anmeldung:
**17.04.2013 Patentblatt 2013/16**

(73) Patentinhaber: **Karl Storz GmbH & Co. KG**
**78532 Tuttlingen (DE)**

(72) Erfinder: **Gloger, Oliver**
**10967 Berlin (DE)**

(74) Vertreter: **Witte, Weller & Partner Patentanwälte mbB**
**Postfach 10 54 62**
**70047 Stuttgart (DE)**

(56) Entgegenhaltungen:
DE-U1-202011 050 001　US-A1- 2005 038 556
US-A1- 2005 119 783　US-A1- 2006 008 866
US-A1- 2009 317 002　US-B1- 6 653 146
US-B2- 7 997 847

• C. Demant, B. Streicher-Abel, A. Springhoff: "Industrielle Bildverarbeitung,", January 2011 (2011-01), Springer, Heidelberg ; 3. Auflage ISBN: 9783642130960 pages 171-194,

EP 2 581 731 B1

**Beschreibung**

Die vorliegende Erfindung betrifft eine Vorrichtung zum Erkennen von Anomalien an medizinischen Instrumenten, mit einer Datenverarbeitungsanlage und einer Instrumenterfassungseinheit bzw. Instrumentenerkennungseinheit (im Folgenden stets Instrumenterfassungseinheit), wobei die Datenverarbeitungsanlage eine Anzeigeeinheit, eine Datenbank, eine Schnittstelle und eine Auswerteeinheit aufweist und die Instrumenterfassungseinheit eine Auflage und zumindest eine Kamera aufweist, wobei die zumindest eine Kamera so angeordnet und ausgerichtet ist, dass sie Bilddaten von auf der Auflage angeordneten medizinischen Instrumenten aus zumindest einer Perspektive erfassen kann, und die Datenverarbeitungsanlage so ausgestaltet ist, dass sie über die erste Schnittstelle Bilddaten von der zumindest einen Kamera erhält und die erhaltenen Bilddaten in der Datenbank speichern und mittels der Auswerteeinheit auf Regionen untersuchen kann, die Anomalien aufweisen. Ferner betrifft die vorliegende Erfindung ein Verfahren zum Erkennen von Anomalien an medizinischen Instrumenten.

Wiederverwendbare medizinische Instrumente sind durch die harschen Bedingungen bei Reinigung und Sterilisation (Einwirkung von Chemikalien, hohe Temperaturen, etc.) und den hohen Turnover häufig materialschädlichen Bedingungen ausgesetzt. Zusätzlich müssen die medizinischen Instrumente wieder schnell einsatzbereit sein, weswegen eine gründliche Untersuchung der Instrumente vor einer Operation, die mit einem hohen Zeitaufwand verbunden ist, kontraproduktiv ist. Da zu einer gründlichen Untersuchung ferner Fachkräfte eingesetzt werden müssen, wirkt sich dies gleichzeitig auch negativ auf die Gesamtkosten aus.

Mögliche Fehler oder Anomalien an solchen Instrumenten können z.B. organische Rückstände von vorhergehenden Operationen sein. Kommen diese bei nachfolgenden Operationen mit den jeweilig folgenden Patienten in Kontakt, kann dies zu Infektionen führen.

Ähnliche Probleme stellen auch Beschädigungen der medizinischen Instrumente dar. Hierfür kommen z.B. Anomalien wie Schäden oder Defekte in Form von Korrosion und/oder Brüchen in Frage. Neben den mit diesen auch verbundenen Infektionsrisiken stellen diese auch eine Gefahr für Verletzungen dar, falls ein solches Instrument beispielsweise in einem Operationsvorgang bricht.

Wenn hier oder im Folgenden von "Anomalie" die Rede ist, ist darunter jeder ungewöhnliche Belag auf dem Instrument oder jede ungewöhnliche Veränderung des Materials des Instruments zu verstehen, der nach außen erkennbar ist, wenn auch nur schwach und möglicherweise für das menschliche Auge nicht sichtbar. Exemplarisch seien hier Verunreinigungen, wie z.B. organische Rückstände oder Rückstände von Reinigungsmitteln, und Schäden des Materials, wie z.B. werkseitige Fehler oder Schäden durch beispielsweise Brüche, Risse, Korrosion, erhebliche, die Funktion beeinträchtigende Kratzer und dgl., genannt.

Infektions- und Verletzungsrisiken der vorgenannten Art sind auf jeden Fall zu vermeiden. Auf der anderen Seite stehen heutzutage immer häufiger Kosten- und Zeitersparnisgründe im Vordergrund. So kommt es gerade im Bereich der Reinigung und Sterilisation von Instrumenten immer öfter dazu, dass Krankenhäuser diese Arbeiten an externe Dienstleister abgeben. Diese sind jedoch nicht immer in der Lage, entweder qualifiziertes Personal einzusetzen oder diesem zumindest die entsprechende Zeit für die notwendigen Kontrollen zu geben, um kosteneffektiv arbeiten zu können. Daraus ergeben sich dann wieder Nachteile für die Qualität der Reinigung und/oder Sterilisation.

Eine Vorrichtung der eingangs genannten Art ist aus der US 7,997,847 B2 bekannt. Die darin beschriebene Vorrichtung dient zur automatisierten Verarbeitung von medizinischen Instrumenten. Darin werden die Instrumente zur Reinigung und/oder zum Verpacken verarbeitet, d.h. insbesondere sortiert. Hierzu ist eine Identifikation der medizinischen Instrumente im System vorgesehen. Dies geschieht durch Aufnahme von Bilddaten mit einer Kamera und Vergleich dieser Bilddaten mit Bilddaten in einer Datenbank. Daraufhin können die Instrumente noch auf mechanische Funktionsfehler durch mechanische Tests und auf das Vorliegen von Verunreinigungen untersucht werden und werden dann in Behältern abgelegt, die dann der Vorrichtung zur weiteren Verwendung entnommen werden können. Die Untersuchung auf Verunreinigungen erfolgt dabei durch einen Abgleich mit Referenzbildern. Diese müssen dafür zunächst in einer langwierigen Prozedur vom System eingelesen und somit hinterlegt werden. Letztendlich wird dann nur eine grobe Aussage ermöglicht, ob das Instrument noch so aussieht, wie es ursprünglich einmal in Form von Bilddaten in einer Datenbank hinterlegt wurde. Eine Identifikation und nähere Analyse der Verunreinigung, d.h. der möglichen Anomalie, erfolgt nicht. Mit anderen Worten wird nur ein Hinweis auf auffällige Regionen vorgenommen. Eine andere Vorrichtung ist aus der DE 20 2011 050 001 U1 bekannt.

Der vorliegenden Erfindung lag daher die Aufgabe zugrunde, eine Vorrichtung und ein Verfahren bereitzustellen, die es ermöglichen, effektiv, schnell, verlässlich und kostensparend Instrumente auf die zuvor genannten Anomalien zu untersuchen und zu überwachen und dabei ferner eine Einordnung der erkannten Anomalien zu ermöglichen.

Diese Aufgabe wird durch eine Vorrichtung gemäß Anspruch 1 gelöst.

Bei solch einer Vorrichtung kann vorteilhafterweise mit Hilfe einer Datenverarbeitungsanlage und einer Bildanalyse eines entsprechenden Instruments schnell eine Untersuchung auf solche Anomalien stattfinden. Diese können sogar ohne größeren Aufwand erkannt werden, wenn es sich bei diesen um kleine, für das menschliche Auge gar nicht oder kaum erkennbare Bereiche handelt. Somit kann z.B. ein feiner Riss vergleichsweise schnell erkannt werden. Auch das Erkennen einer beginnenden anfangs noch kleinen Korrosion ist somit möglich.

Neben der Untersuchung einzelner Instrumente ist im Rahmen dieser Erfindung vorzugsweise auch vorgesehen, dass gleichzeitig mehrere Instrumente auf die entsprechende Auflage gelegt werden und somit simultan von der erfindungsgemäßen Vorrichtung auf Anomalien untersucht werden können. Damit ist eine deutliche Zeitersparnis verbunden. So ist es dadurch z.B. möglich, ein gesamtes Instrumentenset nach dem Zusammenstellen und vor dem Sterilisieren entsprechend zu untersuchen.

Darüber hinaus kann diese Vorrichtung mit anderen Verfahren, die mit Hilfe von Datenverarbeitungsanlagen ablaufen und im Zusammenhang mit der Reinigung, Sterilisation und Organisation solcher medizinischen Instrumente stehen, verbunden werden. Als Beispiel für ein solches Verfahren sei hier die Zusammenstellung von Instrumentensets mit Hilfe von Datenverarbeitungsanlagen erwähnt. Diese kann mit einer Vorrichtung, die einen ähnlichen Aufbau aufweist, stattfinden. So kann dabei auch eine Anordnung der Instrumente auf eine solche Auflage stattfinden, die zu einem solchen Instrumentenset zusammengepackt werden sollen. Bei diesem Auflegen kann die erfindungsgemäße Vorrichtung dann gleichzeitig oder kurz davor bzw. danach eine Untersuchung der jeweiligen Instrumente auf Anomalien durchführen.

Das Speichern der Bilddaten und auch der Analyseergebnisse in der Datenbank hat im Übrigen den Vorteil, dass es bei solch einer Untersuchung der Instrumente mit Hilfe von Datenverarbeitungsanlagen einfach realisierbar ist, sich einen Überblick darüber zu verschaffen, welche Instrumente häufig mit welchen Anomalien beobachtet werden. Durch die so erhaltenen Daten kann ohne größeren Aufwand ein Rückschluss darauf erhalten werden, wo eventuell Fehler im Reinigungs- bzw. Sterilisationsvorgang sind bzw. auch welche Reinigungs- und Sterilisationsvorgänge für welche Instrumente ungeeignet sein könnten. So kann beispielsweise durch wiederkehrendes Auftreten einer Korrosion an mehreren oder allen Instrumenten eines Typs ein Rückschluss darauf gezogen werden, dass z.B. das verwendete Reinigungsmittel zu aggressiv ist. Ferner können auch sogar entdeckte Risse oder Brüche, die z.B. auch durch eine beginnende Korrosion aufgefallen sind, einen Rückschluss auf die Fehlfunktion von Reinigungs- oder Sterilisationsvorrichtungen erlauben. Dies ist beispielsweise dann der Fall, wenn das Instrument immer in der jeweiligen Vorrichtung gegen eine Wand oder ein anderes Bauteil der Vorrichtung oder auch ein anderes Instrument schlägt, z.B. weil eines der beteiligten Instrumente nicht richtig gehalten wird.

Die erfindungsgemäße Ausgestaltung hat ferner den Vorteil, dass sie die Effektivität der Vorrichtung steigert. So kann durch die Einteilung der Anomalien in bestimmte Klassen durch die Datenverarbeitungsanlage schnell eine Zuordnung getroffen werden, ob die erkannte vermeintliche Anomalie, d.h. in erster Linie die Abweichung von der üblichen Instrumentenbeschaffenheit ein regulärer Bestandteil des Instruments oder eine Anomalie im Sinne von Verunreinigung oder Beschädigung ist.

Für den Fall, dass es sich um einen regulären Bestandteil des Instruments handelt, kann die Vorrichtung dies durch Vergleich mit den Klassen für solche Abweichungen durch Instrumentenbestandteile erkennen. Das kann z.B. dann der Fall sein kann, wenn man beispielsweise einen Kunststoffgriff an eigentlich metallenen Instrumenten hat. Die Vorrichtung braucht dann im Übrigen keinen Fehler zu melden bzw. weitere Maßnahmen zu treffen. Für den Fall, dass es sich um eine Verunreinigung oder Beschädigung handelt, kann dann eine entsprechende Fehlermeldung an einen Benutzer erfolgen oder dies beispielsweise zunächst für eine spätere Auswertung gespeichert werden.

Die entsprechend benötigten Klassen, die die erfindungsgemäße Vorrichtung, insbesondere die Datenverarbeitungsanlage für die Vergleiche mit den klassifizierten Anomalien heranzieht, können einerseits herstellerseitig im System vorhanden sein. Vorzugsweise findet das Anlegen der Klassen jedoch für jede Gruppe von Instrumenten individuell statt, um eine möglichst hohe Erfolgsrate durch die erfindungsgemäße Vorrichtung zu erreichen. Dazu weist die erfindungsgemäße Vorrichtung den Benutzer beim Auftreten bisher unbekannter und nicht klassifizierter Anomalien auf diese hin und ermöglicht die Einteilung bzw. Zuordnung der Anomalien zu einer Schaden- bzw. Verunreinigungsart, d.h. zu einer Klasse von Anomalien. Alternativ kann hier dem System dann auch mitgeteilt werden, dass es sich um einen Bestandteil des Instruments und keine Schaden bzw. Verunreinigungen aufweisende Anomalie handelt.

Diese zuvor beschriebene Art des Lernens der Vorrichtung hat den Vorteil, dass selbst bei Einfügen neuer Instrumente, die bisher nicht verwendet wurden und somit unbekannten Typs sind, die Vorrichtung sich stetig an Veränderungen und neu hinzukommende Instrumente und Instrumentenarten anpassen und weiter lernen kann.

Im einfachsten Fall kann die Vorrichtung jedoch ohne weiteres Lernen auch neue oder unbekannte Instrumente untersuchen. Dies liegt daran, dass die vermeintlichen Anomalien, also die Schäden, Verunreinigungen und auch die Instrumentenbestandteile, klassifiziert und in der Datenverarbeitungsanlage hinterlegt werden. Es findet somit vorzugsweise kein Vergleich mit Referenzbildern aller möglichen Instrumente statt, bei dem z.B. Fehler dadurch erkannt werden, dass ein Ist-Zustand von einem Soll-Zustand abweicht. Dies würde im Gegensatz zur vorliegenden Erfindung ein Hinterlegen für jedes verwendete Instrument und für jede seiner Unterklassen und Größen erforderlich machen. Die Erkennung von und Untersuchung auf Anomalien anhand der Klassen von diesen funktioniert somit unabhängig von den tatsächlich vorhandenen Instrumenten, insbesondere von deren genauen Typen und Größen. Dies ist insbesondere dann gegeben, wenn die Instrumente aus den gleichen Werkstoffen, wie z.B. Medizinstahl, gefertigt sind.

In einer weiteren Ausgestaltung der erfindungsgemäßen Vorrichtung ist die Datenverarbeitungsanlage ferner so ausgestaltet, dass sie anhand der Klassifikation die Art der Anomalie angeben kann.

Handelt es sich um einen Schaden bzw. eine Verunreinigung aufweisende Anomalie, kann im Folgenden durch Bestimmung der Klasse eine Zuordnung zu der Art der Anomalie stattfinden. Die Vorrichtung vermag in einem solchen Fall dann entweder dem Benutzer die Art der Anomalie mitzuteilen oder sogar gleich entsprechende Maßnahmen zu treffen. Mit Maßnahmen ist in diesem Zusammenhang z.B. das automatische Aussortieren durch die Vorrichtung gemeint, bei der ein beschädigtes Instrument beispielsweise komplett ausgesondert oder zur Reparatur aussortiert wird. Im Falle einer Verunreinigung würde z.B. dahingehend aussortiert werden, dass das betroffene Instrument einem weiteren Reinigungs- und Sterilisationszyklus unterzogen wird.

In einer weiteren Ausgestaltung der Erfindung ist die Datenverarbeitungsanlage ferner so ausgestaltet, dass das Untersuchen auf und Auffinden von Anomalien anhand der Farbinformation der Bilddaten geschieht.

Diese Ausgestaltung hat den Vorteil, dass damit die Untersuchung anhand einer gut aus den Bilddaten zugänglichen Eigenschaft stattfinden kann. Bedingt durch die Beschaffenheit der medizinischen Instrumente mit ihrer im Allgemeinen metallischen Oberfläche und schwarzen Kunststoffteilen, stellen farbliche Veränderungen gut und schnell erkennbare Abweichungen bzw. Anomalien dar. Auf rechen- und zeitintensive Art von Objekterkennungen als eine alternative Ausgestaltung zur Erkennung von Anomalien kann somit verzichtet werden.

In einer weiteren Ausgestaltung der erfindungsgemäßen Vorrichtung weist die Auflage einen kontrastverstärkenden Hintergrund auf.

Diese Ausgestaltung hat den Vorteil, dass so die Auswerteeinheit der Datenverarbeitungsanlage mit einer deutlich höheren Erfolgsquote und Effektivität das zu untersuchende Instrument von dem Hintergrund unterscheiden kann. Die Farb- und Bildinformationen des Hintergrunds können auf einfache Weise von den relevanten Informationen getrennt werden.

In einer weiteren Ausgestaltung der Erfindung ist die Kamera in ihrer Position veränderbar angeordnet, so dass sie Bilddaten aus zumindest zwei Perspektiven erfassen kann.

Wenn hierbei und im Folgenden von "Perspektive" die Rede ist, so ist hierunter die Orientierung einer jeweiligen Kamera zu einem jeweiligen Instrument in Hinblick auf Azimutwinkel und Polarwinkel gemeint. Dabei führt eine Änderung der Perspektive zu einer Änderung dieser Orientierung, indem zumindest einer dieser beiden Winkel, die zusammen mit dem Abstand zum Ursprung eines Kugelkoordinatensystems die Position eines Objekts in einem solchen System eindeutig festlegen, verändert wird.

Diese Ausgestaltung hat den Vorteil, dass die Erfassung der Bilddaten von den Instrumenten nicht nur aus einer einzigen fixen Perspektive möglich ist. Dies hat in der Folge den Vorteil, dass insbesondere bei komplexeren Instrumenten auch diese von mehreren Seiten auf Anomalien untersucht werden können.

Vorzugsweise ist die Kamera dazu in ihrer Position motorgetrieben veränderbar, noch weiter bevorzugt automatisch veränderbar, indem eine Ansteuerung der Motoren durch die Datenverarbeitungsanlage erfolgen kann.

Dies hat im Weiteren den Vorteil, dass die Erfassung der Bilddaten aus mehreren Perspektiven automatisch in einem Durchgang erfolgen kann und die Datenverarbeitungsanlage so schnell die Untersuchung auf Anomalien ohne weitere Einwirkung eines Benutzers durchführen und abschließen kann.

In einer weiteren Ausgestaltung der erfindungsgemäßen Vorrichtung weist die Auflage eine transparente Grundfläche auf und ist der kontrastverstärkende Hintergrund in seiner Position ober- und unterhalb der beiden Seiten der transparenten Grundfläche veränderbar.

Diese Ausgestaltung hat insbesondere den Vorteil, dass somit ein medizinisches Instrument, welches auf der transparenten Grundfläche angeordnet ist, zum einen von oben als auch von unten in Bezug auf die Bilddaten erfasst werden kann.

Hierzu ist es lediglich notwendig, den kontrastverstärkenden Hintergrund zum einen unterhalb der transparenten Grundfläche und in dem nächsten Schritt oberhalb der transparenten Grundfläche anzuordnen. Mit anderen Worten wird der kontrastverstärkende Hintergrund immer auf der Rückseite des zu erfassenden medizinischen Instruments mit Bezug auf die Kameraperspektive angeordnet.

Mit einer in der Kameraperspektive veränderbar positionierbaren Kamera ist es dann möglich, auch, ggf. motorgetrieben und automatisiert, Bilddaten von unterhalb der transparenten Grundfläche von dem medizinischen Instrument zu erfassen.

Hierzu ist vorzugsweise der kontrastverstärkende Hintergrund automatisch veränderbar angeordnet.

In einer weiteren Ausgestaltung der Erfindung weist die Auflage eine transparente Grundfläche und die Vorrichtung zumindest zwei kontrastverstärkende Hintergründe auf, die jeweils so auf den gegenüberliegenden Seiten der transparenten Grundfläche angeordnet sind, dass jeweils zumindest ein kontrastverstärkender Hintergrund aus einer jeweiligen Kameraperspektive hinter dem zu erkennenden Instrument liegt.

Bei dieser Ausgestaltung wird ein jeweiliger kontrastverstärkender Hintergrund auf jeder Seite der transparenten Grundfläche bereitgestellt. Somit wird für jede Kameraperspektive, unabhängig von der momentanen Anordnung der Kamera mit Bezug auf die Grundfläche, die gute Erkennbarkeit der Instrumente in der Objekterkennung, wie sie zuvor beschrieben wurde, gewährleistet. Dies ist insbesondere bei der Verwendung von zwei Kameras, die jeweils auf einer Seite der Grundfläche angeordnet sind, von Vorteil, da so in einem kurzen zeitlichen Abstand, vorzugsweise gleichzeitig, mit beiden Kameras Aufnahmen gemacht werden können. Damit wird die gesamte Objekterkennung durch die zusätzlichen Bildinformationen optimiert, gleichzeitig aber nicht verlangsamt.

In einer weiteren Alternative weist die erfindungsgemäße Vorrichtung vorzugsweise zumindest eine weitere Kamera auf, wobei zumindest je eine Kamera auf einer Seite der transparenten Grundfläche angeordnet ist und so angeordnet und ausgerichtet ist, dass sie Bilddaten von den auf der Auflage angeordneten Instrumenten aus zumindest einer Perspektive erfassen kann, wobei die Perspektive der einen Kamera von oberhalb und die der weiteren Kamera von unterhalb der transparenten Grundfläche auf die Auflage gerichtet ist.

Diese Ausgestaltung hat dann den Vorteil, dass somit, insbesondere in Kombination mit der zusätzlichen automatisch veränderbaren Positionierbarkeit der jeweiligen Kamera, komplett automatisiert von allen möglichen Perspektiven Bilddaten der jeweiligen Instrumente erfasst werden können. Dadurch können sodann alle nach außen hin erkennbaren Anomalien der medizinischen Instrumente erfasst werden.

Ferner wird die eingangs genannte Aufgabe durch ein Verfahren gemäß Anspruch 7 gelöst.

Dieses Verfahren hat entgegen der bisher üblichen Verfahrenweise den Vorteil, dass der Benutzer nun nicht in mühevoller Arbeit die Instrumente selber absuchen muss, was wie zuvor bereits erwähnt zeit- und kostenintensiv ist, sondern der Benutzer hier lediglich die Instrumente auf die Auflage der Instrumentenerfassungseinheit zu legen braucht. Im Ergebnis gibt die Vorrichtung in diesem Verfahren dann dem Benutzer die Information, ob Anomalien an den Instrumenten entdeckt wurden. Ferner können dem Benutzer weitere Informationen über die Anomalien an sich zur Verfügung gestellt werden, wie z.B. Lage, Art, Größe etc.

Im Übrigen ergeben sich die Vorteile aus den Ausführungen, die zuvor im Zusammenhang mit der Vorrichtung gemacht wurden.

Diese Ausgestaltung des Verfahrens hat den Vorteil, dass durch Auffinden von farblich auffälligen Regionen nach einem Merkmal in den Bilddaten gesucht wird, das charakteristisch für die jeweiligen Anomalien ist. So ist es beispielsweise so, dass Roststellen typischerweise einen vergleichsweise hohen Rotanteil aufweisen, der von der gewöhnlichen Instrumentenfarbe abweicht. Dies wird im vorliegenden Fall zusätzlich dadurch begünstigt, dass die Instrumente metallisch sind und im Übrigen meist nur schwarze Kunststoffteile aufweisen. Sowohl metallisch, also grau-weiß, als auch schwarz unterscheiden sich deutlich von den Farben der Anomalien hinsichtlich der Bilddaten.

Das Klassifizieren und die Bestimmung der Art der Anomalie anhand der Klassifikation hat, wie zuvor bereits ausgeführt, den Vorteil, dass so zum einen einfache Referenzen geschaffen werden können und ferner überdies eine effiziente Methode vorliegt, wie die erkannten Anomalien mit diesen Referenzen verglichen werden können. Hierbei fällt im Übrigen innerhalb dieses Verfahrens nur ein Erlernen der Klassen an. Ein bei Verwenden neuer Instrumente und Instrumententypen notwendiges Hinterlegen von Referenzen für diese ist nicht notwendig, wie zuvor erläutert wurde.

Dies führt im Weiteren, insbesondere in der bevorzugten Ausgestaltung, dazu, dass so auf einfache Weise eine Zuordnung und damit Identifikation der Anomalien möglich ist. Neben der Einordnung der Anomalie entweder als ein regulärer Bestandteil des Instruments oder als eine einen Schaden bzw. eine Verunreinigung aufweisende Anomalie ist somit auch möglich, bei dem letzten Fall eine genaue Zuordnung zu der Art des Schadens bzw. der Verunreinigung zu machen.

Dies kann dann dem Benutzer mitgeteilt werden, so dass dieser weitere Schritte ergreift. Alternativ ist in einer bevorzugten Ausführungsform auch denkbar, dass im Rahmen des erfindungsgemäßen Verfahrens auch gleich eine Aussortierung der mit einer Anomalie erkannten medizinischen Instrumente stattfindet. So können verunreinigte Instrumente zum Zwecke eines weiteren Reinigungszyklus aussortiert werden, während beschädigte und fehlerhafte Instrumente, je nach Art des Schadens, komplett oder zur Reparatur aussortiert werden.

Vorzugsweise werden die Schritte cc) und dd) durch Vergleiche der ermittelten Klasse für die farblich auffällige Region mit bereits bekannten Klassen für Anomalien und Instrumentbestandteile realisiert.

Dies hat den Vorteil, dass hierbei auch auf die gelernten Daten zurückgegriffen wird.

In einer weiteren Ausgestaltung des erfindungsgemäßen Verfahrens weist der Schritt aa) folgende Schritte auf:

- Bestimmen der Farbstärke eines jeden Pixels,

- Vergleich der Farbstärke mit einem vorgegebenen Schwellenwert,

- Einstufen des Pixels als farblich auffällig bei Überschreiten des Schwellenwerts und

- Zusammenfassen aller einander benachbarten farblich auffälligen Pixel zu einer farblich auffälligen Region,

wobei vorzugsweise das Bestimmen der Farbstärke durch Ermittlung der Unterschiedlichkeit der Bildkanalwerte der einzelnen Bildkanäle realisiert wird.

Wenn im Vorhergehenden oder im Folgenden von "Pixel" die Rede ist, so ist hierunter entsprechend der allgemeinen Definition die kleinste Einheit einer digitalen Rastergraphik zu verstehen. Dabei ist davon auszugehen, dass im Rahmen der vorliegenden Erfindung die erhaltenen Bilddaten in Form von Rastergraphiken vorliegen.

Ferner ist so ein Pixel als kleinste Einheit mit Daten versehen, die vorzugsweise Farbinformationen aufweisen. Diese Farbinformationen sind je nach Bilddaten insbesondere je nach deren Format auf einen Farbraum bezogen. Hierbei wird im Rahmen der Beschreibung der vorliegenden Erfindung exemplarisch davon ausgegangen, dass der für die vorhergehenden und folgenden Erläuterungen verwendete Farbraum eines solchen Pixels der RGB-Farbraum ist. Diese Annahme wird jedoch lediglich zum Zwecke der vereinfachten Erläuterung gemacht, um nicht die in Bezug auf einen Farbraum gemachten Äußerungen jeweils für alle möglich denkbaren und existenten Farbräume zu wiederholen. Die Verwendung des RGB-Farbraums ist somit in diesem Zusammenhang nicht als Beschränkung zu verstehen. Somit sind auch andere bekannte Farbräume im Rahmen dieser Erfindung denkbar, wie z.B. der XYZ-, I1I2I3-, YUV- oder der YCbCr-Farbraum, um nur einige bekannte und etablierte Farbräume zu nennen. Daneben ist auch denkbar, dass eigens für das vorliegende Verfahren ein eigener Farbraum entwickelt bzw. festgelegt wird.

Die zuvor genannte Ausgestaltung des erfindungsgemäßen Verfahrens hat den Vorteil, dass das Merkmal der Farbstärke gut dafür geeignet ist, in einem ersten Schritt zunächst zu überprüfen, ob generell signifikante farbliche Auffälligkeiten vorliegen. Dabei ist der Begriff "Farbstärke" so zu verstehen, dass es sich hierbei um das Vorhandensein farblicher Information jedweder Art handelt, die sich von unfarbigen Informationen, wie Weiß, Grau und Schwarz, abheben. Die Farbstärke ist somit in einem ersten Schritt nicht auf einzelne bestimmte Farben beschränkt. Im Rahmen dieser Erfindung liegt somit eine höhere Farbstärke vor, sobald eine Abweichung von den typischen Instrumentenfarben vorliegt. Diese sind entsprechend der zuvor gemachten Ausführungen metallisch Weiß bis Grau und in der Regel Schwarz für entsprechende Kunststoffteile.

Das Vorsehen eines Schwellenwerts hat ferner den Vorteil, dass das Verfahren so in seiner Empfindlichkeit bzw. Sensitivität an die gewünschten Anforderungen angepasst werden kann. Ein Herabsetzen des Schwellenwerts führt somit zu einer höheren Sensitivität, während ein Erhöhen des Schwellenwerts die Sensitivität verringert. Im letzteren Fall würden so nur deutlich farblich auffällige Regionen erkannt werden.

Das Zusammenfassen aller einander benachbarten farblich auffälligen Pixel zu einer farblich auffälligen Region hat im Weiteren den Vorteil, dass diese so auf einfache Weise dem Benutzer präsentiert werden kann. Dies geschieht vorzugsweise durch eine Ausgabe auf einer Anzeigeeinheit einer Vorrichtung, auf der dieses Verfahren ausgeführt wird.

Die Bestimmung der Farbstärke anhand der Unterschiedlichkeit der Bildkanalwerte der einzelnen Bildkanäle stellt eine einfache Möglichkeit dar, aus den vorhandenen Farbinformationen eines Pixels ohne komplexe Berechnungen Informationen darüber zu erhalten, ob der entsprechende Pixel zu einer farblich auffälligen Region gehört oder nicht. Das liegt daran, dass gerade für die oben genannten Farben Weiß, Grau und Schwarz alle Bildkanalwerte eines Pixels nahe beieinander liegen, während bei anderen Farben zumindest ein Bildkanalwert eines Pixels von den anderen Bildkanalwerten deutlich abweicht. Auf diese Weise kann somit eine Abweichung von den genannten typischen Instrumentenfarben erkannt werden.

In einer weiteren bevorzugten Ausgestaltung des erfindungsgemäßen Verfahrens weist der Schritt bb) folgende Schritte auf:

- Ermitteln der Wahrscheinlichkeit für jedes Pixel einer farblich auffälligen Region, zu einer bestimmten Klasse von Anomalien zu gehören,

- Auswählen und Zuweisen der Klasse mit der größten Wahrscheinlichkeit zu dem jeweiligen Pixel, und

- Auswählen der am häufigsten auftretenden Klasse bei der Gesamtheit aller Pixel der farblich auffälligen Region und Zuweisen dieser Klasse zu der farblich auffälligen Region.

Das Ermitteln und Zuweisen einer bestimmten Klasse zu einem jeweiligen Pixel anhand der Wahrscheinlichkeiten hat den bedeutenden Vorteil, dass das erfindungsgemäße Verfahren so lernfähig ist. Dies ergibt sich daraus, dass die Wahrscheinlichkeiten nicht fix, sondern variabel und veränderbar sind. Dies wird vorzugsweise in der Form realisiert, dass entweder zunächst anfangs als auch jederzeit während des Betriebs einer Vorrichtung mit dem erfindungsgemäßen Verfahren ein separates Lernverfahren möglich ist. In diesem Lernverfahren werden dann entsprechend Bilddaten von anomalienbehafteten medizinischen Instrumenten erfasst. Diese Bilddaten werden auf Anomalien entsprechend dem erfindungsgemäßen Verfahren untersucht. Wird dabei eine Anomalie entdeckt, kann der Benutzer diese Anomalie einer bestimmten Klasse, entweder von regulären Instrumentbestandteilen oder Schäden bzw. Verunreinigungen, zuweisen. Basierend auf diesen Daten werden dann Wahrscheinlichkeiten ermittelt, wonach für jedes Pixel anhand der Farbinformation und in Bezug auf jede vorhandene Klasse eine Wahrscheinlichkeit ermittelt wird, mit der die ermittelte Farbinformation zu den jeweiligen Klassen gehört.

Neben einer expliziten Lernphase ist ferner auch ein Eingriff durch den Benutzer bei Ausführung des erfindungsgemäßen Verfahrens jederzeit denkbar und möglich, wenn dieser erkennt, dass eine Zuordnung zu einer Klasse nicht korrekt ist. Hierdurch werden dann auch die entsprechenden Wahrscheinlichkeitswerte angepasst.

Die verbleibenden Schritte mit dem Auswählen und Zuweisen der Klasse mit der größten Wahrscheinlichkeit zu dem jeweiligen Pixel und dem Auswählen der am häufigsten auftretenden Klasse unter den Pixeln der farblich auffälligen Region und dem Zuweisen dieser Klasse zu der farblich auffälligen Region führt dann in vorteilhafter Weise dazu, dass der auffälligen Region letztendlich die Klasse zugewiesen wird, der die Ursache für die farbliche Auffälligkeit angehört.

Ferner ist Gegenstand ein Computerprogramm, das Programmcodebestandteile aufweist, die auf einem Computer ausgeführt dazu führen, dass der Computer die Schritte des erfindungsgemäßen Verfahrens entsprechend einer der zuvor genannten Ausgestaltungen durchführt.

In diesem Zusammenhang ist ferner auch ein Computer-lesbares nicht vorübergehendes Medium Gegenstand dieser Erfindung, auf dem Anweisungen gespeichert sind, die, wenn sie auf einem Computer ausgeführt werden, den Computer dazu bringen, dass er die Schritte des erfindungsgemäßen Verfahrens entsprechend einer der zuvor genannten Ausgestaltungen durchführt.

Ein Computerprogramm kann auf einem geeigneten Medium gespeichert/vertrieben werden, wie z.B. einem optischen Speichermedium oder einem festen Speichermedium, dass zusammen mit oder als Teil von anderer Hardware geliefert wird. Ferner kann es aber auf andere Weise vertrieben werden, wie z.B. über das Internet oder auf anderen kabelgebundenen oder kabellosen Telekommunikationswegen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:

Fig. 1     eine schematische perspektivische Darstellung einer erfindungsgemäßen Vorrichtung mit einer Instrumenterfassungseinheit,

Fig. 2     eine schematische Darstellung des Aufbaus einer erfindungsgemäßen Datenverarbeitungsanlage,

Fig. 3     eine schematische perspektivische Darstellung einer weiteren erfindungsgemäßen Vorrichtung mit einer Kombination aus Instrumenterfassungseinheit und Groberkennungseinheit, Letztere mit einer Kamera,

Fig. 4     eine schematische perspektivische Darstellung einer weiteren erfindungsgemäßen Vorrichtung mit einer Kombination aus Instrumenterfassungseinheit mit Groberkennungseinheit, Letztere mit einer Waage,

Fig. 5     eine schematische Darstellung einer Datenverarbeitungsanlage für eine Vorrichtung entsprechend der Fig. 4,

Fig. 6     eine schematische perspektivische Darstellung einer Groberkennungseinheit mit Kamera und Waage,

Fig. 7     eine schematische Darstellung einer Datenverarbeitungsanlage für eine Gesamtvorrichtung mit einer Groberkennungseinheit entsprechend Fig. 6,

Fig. 8     eine schematische perspektivische Darstellung einer Kameraanordnung für Instrument- und Groberkennungseinheiten mit veränderbarer Perspektive,

Fig. 9     eine schematische Seitendarstellung einer erfindungsgemäßen Vorrichtung mit Instrumenterfassungseinheit mit erster und zweiter Kamera und kontrastverstärktem Hintergrund unter dem zu erkennenden Instrument,

Fig. 10     eine schematische Seitendarstellung einer erfindungsgemäßen Vorrichtung mit Instrumenterfassungseinheit mit erster und zweiter Kamera entsprechend der Fig. 9, mit kontrastverstärkendem Hintergrund über dem zu erkennenden Instrument,

Fig. 11     eine schematische Seitendarstellung einer weiteren Ausführungsform einer erfindungsgemäßen Vorrichtung mit einer Instrumenterfassungseinheit analog zur Vorrichtung aus den Fig. 9 und 10 und zwei kontrastverstärkenden Hintergründen,

Fig. 12     eine schematische Seitendarstellung einer weiteren Ausführungsform einer erfindungsgemäßen Vorrichtung mit einer Instrumenterfassungseinheit analog zur Vorrichtung aus den Fig. 9, 10 und 11, mit ebenfalls zwei kontrastverstärkenden Hintergründen,

Fig. 13      eine vergrößerte schematische ausschnittsweise Darstellung eines medizinischen Instruments mit einer Anomalie,

Fig. 14      eine stark vergrößerte schematische Darstellung des Kreises A aus Fig. 13 und

Fig. 15      eine stark vergrößerte schematische Darstellung des Kreises B aus Fig. 13.

Die im Nachfolgenden gezeigten und beschriebenen erfindungsgemäßen Vorrichtungen sind in ihrer Gesamtheit mit den Bezugsziffern 10, 100, 150, 300, 350 und 370 bezeichnet.

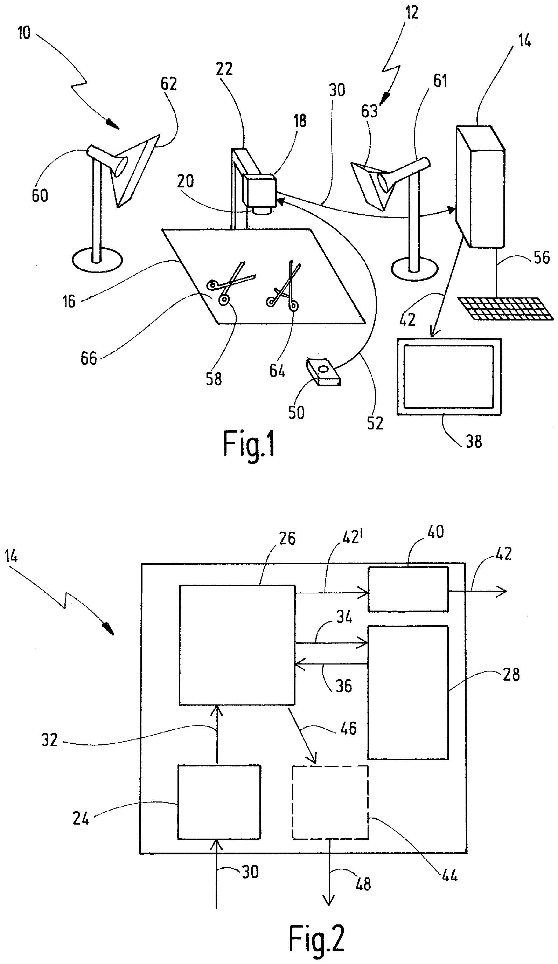

Die in Fig. 1 gezeigte erfindungsgemäße Vorrichtung 10 weist eine Instrumenterfassungseinheit 12 und eine Datenverarbeitungsanlage 14 auf. Die Instrumenterfassungseinheit 12 weist eine Auflage 16 und eine erste Kamera 18 auf.

Die erste Kamera 18 ist so über der Auflage 16 angeordnet, dass ihr Objektiv 20, und entsprechend somit ihre Perspektive auf die Auflage 16 gerichtet ist. Hierzu ist die erste Kamera 18 mittels eines Stativs 22 über der Auflage 16 mit Bezug auf die Darstellung von Fig. 1 angeordnet.

Die Datenverarbeitungsanlage 14 weist ihrerseits eine erste Schnittstelle 24, eine Auswerteeinheit 26 sowie eine Datenbank 28 auf, wie dies in Fig. 2 zu sehen ist. Über die erste Schnittstelle 24 ist die Datenverarbeitungsanlage 14 mit der ersten Kamera 18 verbunden und erhält über diese erste Schnittstelle 24 Bilddaten von der ersten Kamera 18. Dies ist durch einen Pfeil 30 schematisch in Fig. 1 und 2 angedeutet. Die so erhaltenen Bilddaten werden dann von der ersten Schnittstelle 24 an die Auswerteeinheit 26 weitergegeben. Dies ist durch einen Pfeil 32 schematisch angedeutet. Die Auswerteeinheit 26 kann nun mit der Auswertung und Analyse der so erhaltenen Bilddaten direkt beginnen, oder die Daten zunächst in der Datenbank 28 ablegen. Dies ist durch einen Pfeil 34 angedeutet. Zum Zwecke der Auswertung der Daten in der Auswerteeinheit 26 benötigt diese Informationen zu Klassen von Anomalien, wie dies im Folgenden noch näher beschrieben wird. Diese erhält die Auswerteeinheit 26 ebenfalls von der Datenbank 28, wie dies durch einen Pfeil 36 schematisch angedeutet ist.

Hat die Auswerteeinheit 26 ein Auswerteergebnis ermittelt, wird dieses an eine Anzeigeeinheit 38 weitergegeben. Dies kann über einen separaten Anschluss 40 der Datenverarbeitungsanlage 14 geschehen und ist im Übrigen schematisch durch Pfeile 42 und 42' angedeutet. Für eine solche Anzeigeeinheit 38 kommen unterschiedliche Ausgestaltungsmöglichkeiten in Betracht. So ist es zum einen denkbar, spezielle Anzeigeeinheiten für den vorgesehen Zweck zu konstruieren und in die Datenverarbeitungsanlage 14 zu integrieren. Im Übrigen ist es aber auch denkbar, einen gewöhnlichen Computermonitor hierzu zu verwenden, der über den Anschluss 40 ansteuerbar ist.

Neben oder zusätzlich zu der beschriebenen Möglichkeit der Ausgabe der Auswertedaten auf der Anzeigeeinheit 38 ist es auch denkbar, dass die Datenverarbeitungsanlage 14 ferner eine Steuereinheit 44 aufweist. Diese ist in der Fig. 2 mit gestrichelten Linien als optionales Element dargestellt. Die Steuereinheit 44 kann Daten von der Auswerteeinheit 26 erhalten, wie dies schematisch durch einen Pfeil 46 angedeutet ist, und diese dann dazu nutzen, externe Geräte oder Vorrichtungen direkt oder mittelbar anzusteuern. Dies ist durch einen Pfeil 48 ebenfalls schematisch angedeutet. Beispiele für solche Vorrichtungen sind Roboter, die hier aber nicht näher gezeigt sind.

Um ein Auslösen der Bilderfassung der ersten Kamera 18 zu erreichen, ist in der Vorrichtung 10 ferner ein Taster 50 vorgesehen. Dieser Taster 50 ist funktional mit der ersten Kamera 18 verbunden. Dies ist schematisch mit Hilfe eines Pfeils 52 angedeutet. Um im Übrigen eine weitere Kommunikation zwischen einem Benutzer und der Vorrichtung 10 zu ermöglichen, weist diese ferner eine Tastatur 54 auf, die mit der Datenverarbeitungsanlage 14 verbunden ist. Dies ist schematisch mit einer Verbindungslinie 56 angedeutet.

Ein zu untersuchendes medizinisches Instrument ist in der Fig. 1 schematisch als Schere 58 dargestellt. Diese Schere 58 ist auf der ersten Auflage 16 angeordnet. Damit befindet sich die Schere 58 unterhalb der ersten Kamera 18. Entsprechend der perspektivischen Ausrichtung dieser ersten Kamera 18 wird diese Schere 58 bei einer Erfassung der Bilddaten durch die erste Kamera 18 erfasst.

Um die Bildaufnahmen bzw. die Bilderfassung zu optimieren, weist die Vorrichtung 10 ferner Beleuchtungseinrichtungen auf. Diese Beleuchtungseinrichtungen sind hier schematisch durch Lampen 60 und 61 dargestellt. Um ferner Reflektionen an den zu erkennenden Instrumenten zu vermeiden, sind ferner Elemente 62 und 63 für diffuse Lichtverhältnisse vorgesehen. Diese Elemente 62 und 63 können z.B. aus Spezialfotokartonpapier hergestellt sein.

Die Funktionsweise der Instrumenterfassungseinheit 12 hinsichtlich der Erfassung der Bilddaten soll nun exemplarisch anhand der Vorrichtung 10 und der Fig. 1 und 2 erläutert werden.

Zunächst legt ein Benutzer die zu untersuchenden Instrumente auf die Auflage 16. Diese sind hier exemplarisch anhand der Schere 58 und einer Klemme 64 dargestellt.

Nach erfolgtem Auflegen der Instrumente auf die Auflage 16 werden die Bilddaten dieser Instrumente mit der ersten Kamera 18 erfasst. Dies kann in einer bevorzugten Ausführungsform durch Betätigen des Tasters 50 durch den Benutzer ausgelöst werden. Alternativ ist auch eine Auslösung durch den Benutzer mit Hilfe der Tastatur 54 denkbar.

Die durch die erste Kamera 18 erfassten Bilddaten werden nun über die erste Schnittstelle 24 an die Auswerteeinheit 26 der Datenverarbeitungsanlage 14 weitergegeben. Dort werden diese dann in der Auswerteeinheit 26 weiter verarbeitet und analysiert, wie im Folgenden noch näher beschrieben wird.

Das Ergebnis der Untersuchung wird dann auf der Anzeigeeinheit 38 ausgegeben.

In diesem Zusammenhang sei erwähnt, dass es für die korrekte Untersuchung der Instrumente auf der Auflage 16 sinnvoll ist, die Instrumente so anzuordnen, dass sie einander nicht überlagern.

Um die Erfassung der Bilddaten möglichst optimal zu gestalten, so dass die entsprechende Untersuchung möglichst gute Ergebnisse liefert, ist es ratsam für gute Beleuchtungs- und Kontrastverhältnisse zu sorgen.

Hierzu kann die Auflage 16 mit einem kontrastverstärkenden Hintergrund 66 ausgestaltet sein. Dies ist in einfacher Weise beispielsweise dadurch zu realisieren, dass die Auflage 16 einen gleichmäßigen Farbton, z.B. blau, aufweist.

Ferner sollten die Lampen 60 und 61 eine starke Beleuchtung der Auflage 16 liefern und durch die Elemente 62 und 63 gleichzeitig für möglichst diffuses Licht gesorgt werden, um unerwünschte Reflektionen zu vermindern.

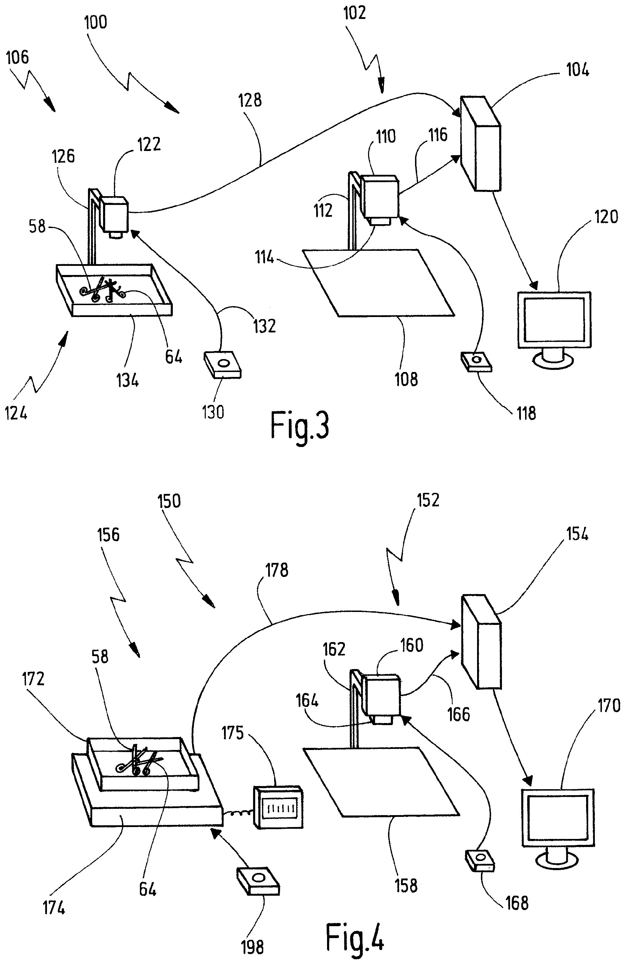

In Fig. 3 ist eine weitere erfindungsgemäße Vorrichtung 100 gezeigt.

Diese Vorrichtung 100 weist eine Instrumenterfassungseinheit 102, eine Datenverarbeitungsanlage 104 sowie eine Groberkennungseinheit 106 auf.

Die Datenverarbeitungsanlage 104 ist vom Aufbau ausgestaltet wie die Datenverarbeitungsanlage 14 und im Folgenden nicht näher gezeigt. Vielmehr wird auf die vorherigen Ausführungen verwiesen. Die Instrumenterfassungseinheit 102 weist eine Auflage 108 und eine erste Kamera 110 auf. Die erste Kamera 110 ist dabei an einem Stativ 112 angeordnet. Dadurch kann sie so ausgerichtet werden, dass ihr Objektiv 114 auf die Auflage 108 ausgerichtet ist. Die hier gezeigte erste Kamera 110 hat somit die gleiche Perspektive wie die Kamera 18 aus Fig. 1.

Die erste Kamera 110 gibt ebenfalls die erfassten Bilddaten an die Datenverarbeitungsanlage 104 weiter, wie dies durch den Pfeil 116 angedeutet ist. Die Erfassung der Bilddaten kann in gleicher Weise wie bei der Vorrichtung 10 aus Fig. 1 durch einen Taster 118 ausgelöst werden. Zur weiteren Kommunikation zwischen dem Benutzer und der Vorrichtung 100 weist die Datenverarbeitungsanlage 104 ferner eine Anzeigeeinheit 120 auf. Der übrige Aufbau und die Funktionsweise der Instrumenterfassungseinheit 102 zusammen mit der Datenverarbeitungsanlage 104 sind vom Prinzip identisch mit dem Aufbau und der Funktionsweise der Instrumenterfassungseinheit 12 zusammen mit der Datenverarbeitungsanlage 14 der Vorrichtung 10, weswegen hierzu auch auf die zuvor gemachten Erläuterungen verwiesen wird.

Zum Zwecke der besseren Ausleuchtung und der Optimierung des Kontrastverhältnisses zwischen aufgelegten Instrumenten auf der ersten Auflage 108 kann hier ebenfalls ein kontrastverstärkender Hintergrund verwendet werden, wie auch starke Beleuchtungsmittel eingesetzt werden, die hier zum Zwecke der Übersichtlichkeit nicht näher gezeigt sind.

Die zusätzliche Groberkennungseinheit 106 der Vorrichtung 100 weist eine weitere Kamera 122 auf. Diese weitere Kamera 122 ist analog zu den zuvor beschriebenen ersten Kameras 110 und 18 über einer zweiten Auflage 124 angeordnet. Hierzu ist sie an einem Stativ 126 befestigt. Die weitere Kamera 122 ist ebenfalls mit der Datenverarbeitungsanlage 104 über eine hier nicht näher gezeigte dritte Schnittstelle verbunden. Dies ist schematisch durch den Pfeil 128 angedeutet. Um auch bei der Groberkennungseinheit 106 eine Bilddatenerfassung durch den Benutzer steuern lassen zu können, weist die Groberkennungseinheit 106 ebenfalls einen Taster 130 auf. Der Taster 130 steht zur Ansteuerung funktional mit der Kamera 122 in Verbindung. Dies ist durch einen Pfeil 132 schematisch angedeutet.

Die Funktionsweise der Vorrichtung 100 ähnelt zunächst der Funktionsweise der Vorrichtung 10. Auch hier werden zunächst durch einen Benutzer entsprechende zu untersuchende Instrumente auf die erste Auflage 108 aufgelegt und entsprechend der zuvor gemachten Ausführungen im Zusammenhang mit der Instrumenterfassungseinheit 12 und der Datenverarbeitungsanlage 14 durch die Instrumenterfassungseinheit 102 und Datenverarbeitungsanlage 104 auf Anomalien untersucht. Daraufhin kann der Benutzer dann die Instrumente auf die zweite Auflage 124 überführen, die beispielsweise keine Anomalien aufweisen. Der daraus folgende Zustand ist in der Fig. 3 schematisch wieder durch die Instrumente der Fig. 1, nämlich die Schere 58 und die Klemme 64 angedeutet.

Liegen nun alle Instrumente auf der zweiten Auflage 124, kann die weitere Kamera 122 mit der Bilderfassung beginnen. Dies kann vorzugsweise mit Hilfe des Tasters 130 durch den Benutzer ausgelöst werden. Die so ermittelten Bilddaten werden dann wie dies durch den Pfeil 128 dargestellt ist, an die Datenverarbeitungsanlage 104 weitergegeben, die dann über ihre Auswerteeinheit mit der Auswertung beginnen kann. Hierbei kann dann beispielsweise die Vollständigkeit eines Instrumentensets anhand einer in der Datenverarbeitungsanlage 104 hinterlegten Packliste überprüft werden.

In einer bevorzugten Ausführungsform weist die zweite Auflage 124 ein Instrumentensieb 134 auf. In dieses Instrumentensieb 134 können dann die Instrumente des Instrumentensets, also hier die Schere 58 und Klemme 64 direkt gelegt werden, wenn sie von der Instrumenterfassungseinheit 102 überführt werden. Dies hat den Vorteil, dass nach erfolgreicher Groberkennung das Instrumentensieb 134 aus der Groberkennungseinheit 106 entnommen und so direkt zur Reinigung bzw. Sterilisation überbracht werden kann.

Da der zur Verfügung stehende Platz in einem solchen Instrumentensieb 134 in der Regel geringer ist als er z.B. auf der ersten Auflage 108 bzw. 16 vorliegt, kommt es in der Groberkennungseinheit häufig zur Überlagerung der zu erkennenden Instrumente, wie dies auch in der Fig. 3 für die Schere 58 und Klemme 64 angedeutet ist.

Um dennoch eine effiziente Möglichkeit zur Erkennung der Vollständigkeit des Instrumentensets zu liefern, wird hierbei vorzugsweise die Erkennung von Schlüsselmerkmalen in den Bilddaten durchgeführt. Diese Schlüsselmerkmale können beispielsweise vorher durch das System erlernt oder direkt vom Benutzer vorgegeben worden sein.

Ein weiterer Vorteil der Verwendung von Schlüsselmerkmalen zur Erkennung der Instrumente ist dass der nachfolgende Groberkennungsschritt durch die Groberkennungseinheit 106 vergleichsweise wenig Zeit beansprucht.

Unter dem Begriff "Schlüsselmerkmale" sind im Rahmen dieser Erfindung optische Instrumentenmerkmale zu verstehen. Diese können beim Eintrainieren eines Instruments automatisch vorgegeben oder auch mittels automatischer Algorithmen für jedes Instrument berechnet werden. Hierbei handelt es sich in aller Regel um auffällige, markante und/oder gut sichtbare Bereiche des jeweiligen Instruments. Aus diesem Grund ist es auch möglich, dass insbesondere ein erfahrener Packer selber beim Eintrainieren angibt, welche Instrumentenbereiche Schlüsselmerkmale darstellen, die sie von anderen, sehr ähnlich aufgebauten Instrumenten unterscheiden. Hierbei kann es sich auch um kleine Details, wie z.B. kleine Instrumentenbestandteile, verschiedene Oberflächenriffelungen, Einbuchtungen, kleine Nuten oder dergleichen handeln. Auch extra für diesen Zweck aufgebrachte Markierungen oder andere Kennzeichnungen sind denkbar. Die alternative oder zusätzliche automatische Bestimmung von Schlüsselmerkmalen erfolgt beispielsweise über sogenannte Interest-Operatoren, wie z.B. dem Förstner-Operator oder dem Moravec-Operator.

Durch die Verwendung der Groberkennungseinheit 106 hat die Gesamtvorrichtung 100 den Vorteil, dass das so bestückte Instrumentensieb 134 dahingehend abschließend geprüft wird, ob das Instrumentenset vollständig vorliegt und das Instrumentensieb 134 somit korrekt bestückt wurde. Fehler dergestalt, dass ein Instrument von einer vorherigen Bereitstellung, beispielsweise in der Instrumenterfassungseinheit 102 nicht korrekt in das zugehörige Instrumentensieb 134 überführt wird, werden dadurch ausgeschlossen.

Neben der zuvor beschriebenen und in den folgenden Ausführungen dargestellten Möglichkeit einer separaten Groberkennungseinheit neben einer Instrumenterfassungseinheit, ist es im Rahmen dieser Erfindung ebenfalls denkbar, die Einheiten konstruktiv zusammenzulegen. Damit wären erste und zweite Auflage sowie erste und weitere (oder zweite und weitere) Kamera identisch. Eine solche Anordnung hat einen geringeren Platzbedarf gegenüber einer separaten Anordnung.

Fig. 4 zeigt eine weitere erfindungsgemäße Vorrichtung 150.

Diese erfindungsgemäße Vorrichtung 150 weist ebenfalls eine Instrumenterfassungseinheit 152, eine Datenverarbeitungseinheit 154 und eine Groberkennungseinheit 156 auf.

Die Instrumenterfassungseinheit 152 weist in vergleichbarer Weise zu den Vorrichtungen 10 und 100 eine Auflage 158 sowie eine erste Kamera 160 mit Stativ 162 und Objektiv 164 auf. Auch die erste Kamera 160 steht in Verbindung mit der Datenverarbeitungsanlage 154, wie dies durch einen Pfeil 166 angedeutet ist. Auch die erste Kamera 160 kann durch einen Taster 168 ausgelöst werden.

Die Funktionsweise der Instrumenterfassungseinheit 152 ist analog zu der Instrumenterfassungseinheit 102, weswegen hier auf weitere Ausführungen verzichtet und lediglich auf die vorherigen Ausführungen verwiesen wird. Ein entsprechendes Ergebnis der Untersuchung der Instrumente erfolgt dann mit Bezug auf das zu bestückende Instrumentenset über eine Anzeigeeinheit 170.

Ebenfalls analog zu der Vorrichtung 100 aus Fig. 3 soll anschließend zur Überprüfung der Vollständigkeit eines entsprechenden Instrumentensets eine Groberkennung in der Groberkennungseinheit 156 vorgenommen werden.

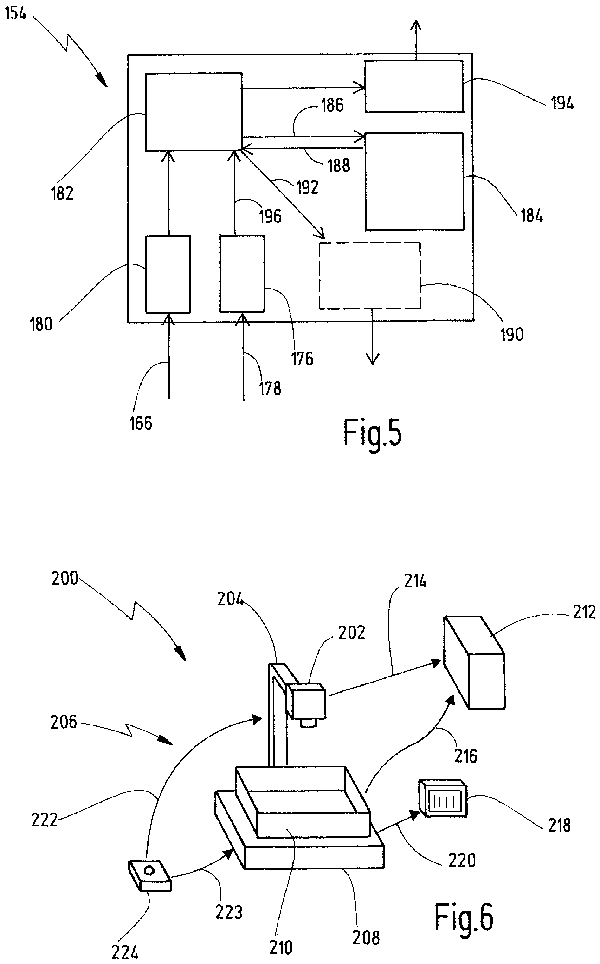

Hierzu weist die Groberkennungseinheit 156 auch in der bevorzugten Ausführungsform ein Instrumentensieb 172 auf. Dieses Instrumentensieb 172 ist auf einer Waage 174 angeordnet. Die Waage 174 weist zum einen eine eigene Anzeigeeinheit 175 auf. Ferner steht die Waage 174 mit der Datenverarbeitungsanlage 154 über eine zweite Schnittstelle 176 in Verbindung. Dies ist durch einen Pfeil 178 angedeutet und auch in Fig. 5 zu erkennen.

Die in Fig. 5 schematisch gezeigte Datenverarbeitungsanlage 154 stimmt im Wesentlichen mit der Datenverarbeitungsanlage 14 der Fig. 2 überein. Auch hierbei gibt es eine erste Schnittstelle 180 zur Aufnahme der Bilddaten aus der ersten Kamera 160. Dies ist schematisch durch einen Pfeil 166 angedeutet. Weiterhin weist die Datenverarbeitungsanlage 154 auch eine Auswerteeinheit 182 sowie eine Datenbank 184 auf. Die Auswerteeinheit 182 kann Daten in die Datenbank 184 schreiben und aus dieser auslesen, wie dies durch Pfeile 186 und 188 angedeutet ist. Im Übrigen weist die Datenverarbeitungsanlage 154 auch eine optionale Steuereinheit 190 auf, die ebenfalls von der Auswerteeinheit 182 mit Daten zur Steuerung weiterer Geräte und Vorrichtungen versorgt werden kann. Dies ist durch einen Pfeil 192 angedeutet. Auch weist die Datenverarbeitungsanlage 154 einen Anschluss 194 auf, über den eine nicht näher gezeigte Verbindung zu der Anzeigeeinheit 170 erfolgen kann.

Als Unterschied zur Datenverarbeitungsanlage 14 weist die Datenverarbeitungsanlage 154 zusätzlich die zweite Schnittstelle 176 auf. Diese dient, wie zuvor bereits beschrieben, zur Weitergabe der Daten der Waage 174 an die Auswerteeinheit 182. Dies ist schematisch durch einen Pfeil 196 angedeutet.

Werden nun wie dies in Fig. 4 angedeutet ist, wieder Instrumente, hier die Schere 58 und die Klemme 64, als fertiges Instrumentenset in das Instrumentensieb 172 gelegt, bestimmt die Waage das Gewicht dieses Instrumentensets.

Dies geschieht vorzugsweise durch Betätigen eines Tasters 198. Mit Gewicht des Instrumentensets ist bevorzugt dessen Gesamtgewicht gemeint. Dies geschieht vorzugsweise nach vorheriger Tarierung der Waage mit dem Instrumentensieb 172, um Ungleichmäßigkeiten in den Gewichten der verwendeten Instrumentensiebe zu kompensieren.

Die so erhaltenen Gewichtsdaten werden dann über die zweite Schnittstelle 176 zur Auswerteeinheit 182 der Datenverarbeitungsanlage 154 weitergegeben. Dort erfolgt ein Vergleich mit dem Sollgewicht des vorliegenden Instrumentensets aus Schere 58 und Klemme 64. Dieses ist als Referenz in der Datenbank 184 hinterlegt. Stimmt das ermittelte Gewicht von Schere 58 und Klemme 64 mit dem hinterlegten Sollgewicht überein, wird von der Datenverarbeitungsanlage 154 eine entsprechende Mitteilung an den Benutzer über die Anzeige 170 gegeben.

Weicht das ermittelte Gewicht jedoch von dem hinterlegten Sollgewicht nach Berücksichtigung eventueller Toleranzen ab, wird dies als Fehler im Instrumentenset an den Benutzer über die Anzeigeeinheit 170 mitgeteilt.

Hieraufhin hat der Benutzer das vermeintlich vollständige Instrumentenset noch einmal zu überprüfen. Mögliche Fehler können hierbei falsche Übertragung der Instrumente von Instrumenterfassungseinheit zu Groberkennungseinheit sein, als auch Fehler insbesondere beim Zusammenbau von komplexeren Instrumenten. So können z.B. einzelne innen liegende Bauteile fehlen. Dies ist nicht von außen zu erkennen und somit nicht für den Benutzer einfach zu ermitteln.

Die Hinterlegung des Referenzgewichts bzw. Sollgewichts eines jeweiligen Instrumentensets in der Datenbank 184 kann unterschiedlich geschehen. Eine Möglichkeit ist hierbei die Hinterlegung der Einzelgewichte der jeweiligen Instrumente. Diese würden dann von der Datenverarbeitungsanlage 154 für jedes entsprechende Instrumentenset jeweils neu berechnet und dann entsprechend mit den ermittelten Gewichten verglichen werden. Alternativ kann auch eine Bestimmung von Sollgewichten kompletter Instrumentensets erfolgen und somit für jedes Instrumentenset als fester Wert in der Datenbank 184 abgelegt werden.

Die Bestimmung der Soll- bzw. Referenzgewichte der Instrumente oder Instrumentensets geschieht entweder durch die Aufnahme von Herstellerdaten in die Datenverarbeitungsanlage 154 oder durch eigene Wägungen in einer Lernphase. Hierbei werden vorzugsweise mehrere Wägevorgänge mit den jeweiligen Instrumenten(sets) vorgenommen, so dass, insbesondere bei unterschiedlichen Umgebungsbedingungen wie Feuchtigkeit und Temperatur, mehrere Gewichte für ein Instrument bzw. Instrumentenset vorliegen. Dadurch kann dann eine entsprechende Fehlertoleranz anhand der Standardabweichung ermittelt und hinterlegt werden.

Neben der zuvor gezeigten Ausgestaltung der Vorrichtung 100 und 150 mit den Groberkennungseinheiten 106 und 156 ist in Fig. 6 eine weitere bevorzugte Ausführungsform in Form einer Groberkennungseinheit 200 gezeigt.

Wenngleich die Groberkennungseinheit 200 hier als einzelnes Element dargestellt ist, so versteht es sich, dass diese in beliebiger Form mit den zuvor gezeigten Instrumenterfassungseinheiten 12, 102 und 152 kombiniert werden kann.

Die Groberkennungseinheit 200 kann als eine Kombination der Groberkennungseinheiten 106 und 156 angesehen werden.

Die Groberkennungseinheit 200 weist eine weitere Kamera 202 auf, die an einem Stativ 204 angeordnet ist. Die Anordnung der weiteren Kamera 202 ist in analoger Weise so, dass sie über einer zweiten Auflage 206 angeordnet und so ausgerichtet ist, dass die Kameraperspektive auf diese zweite Auflage 206 gerichtet ist. Auch die zweite Auflage 206 weist eine Waage 208 auf. Auf dieser Waage 208 ist ein Instrumentensieb 210 angeordnet. Sowohl die weitere Kamera 202 als auch die Waage 208 sind mit einer Datenverarbeitungsanlage 212 verbunden, wie dies schematisch durch Pfeile 214 und 216 angedeutet ist.

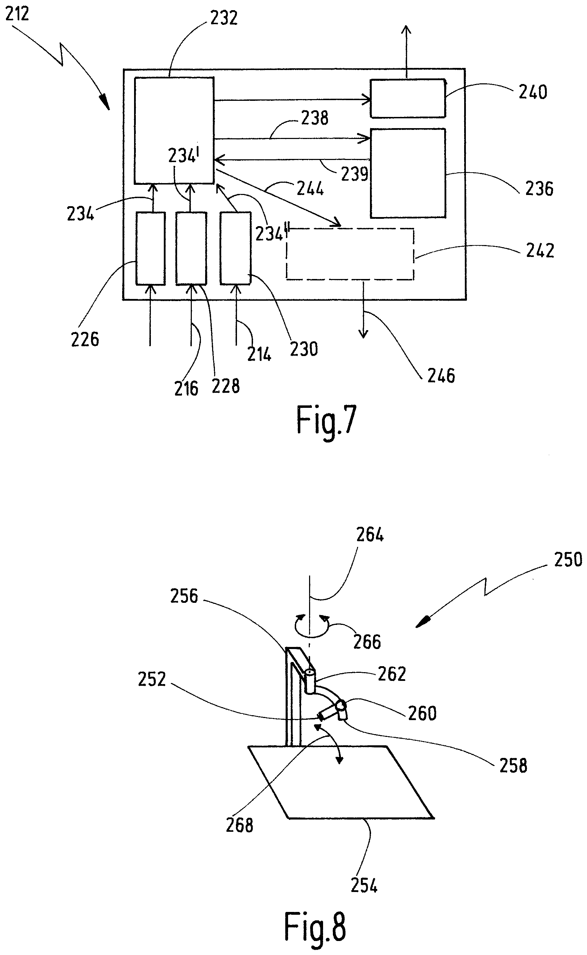

Die Datenverarbeitungsanlage 212 ist im Übrigen analog zu den zuvor beschriebenen Datenverarbeitungsanlagen 14, 104 und 154 ausgestaltet und näher im Zusammenhang mit der Fig. 7 beschrieben. Sie steht im Übrigen in Verbindung mit einer Anzeigeeinheit, die der Übersichtlichkeit halber nicht in der Fig. 6 dargestellt ist.

Zusätzlich kann die Waage noch mit einer eigenen Anzeigeeinheit 218 verbunden sein, wie dies schematisch durch einen Pfeil 220 angedeutet ist. Die Anzeigeeinheit 218 dient dann dazu, das jeweilige Gewicht der auf der Waage 208 angeordneten Gegenstände anzuzeigen.

Um den Bilderfassungs- und Wägevorgang auszulösen, weisen auch hier die weitere Kamera 202 und Waage 208 eine Ansteuerung durch Taster auf, wie dies durch Pfeile 222 und 223 schematisch angedeutet ist. Dies kann durch separate einzelne Taster geschehen oder, wie hier in Fig. 6 dargestellt, durch einen gemeinsamen Taster 224 erfolgen.

Die Groberkennung mit der Groberkennungseinheit 200 läuft im Wesentlichen analog zu den zuvor beschriebenen Groberkennung mit den Groberkennungseinheiten 106 und 156 ab. Unterschied zu der zuvor beschriebenen Groberkennung ist hierbei jedoch die Möglichkeit zur simultanen Erkennung mittels der weiteren Kamera 202 und der Waage 208.

Diese Variante hat den Vorteil, dass zum einen Fehler beim Zusammenbau von komplexeren Instrumenten aufgrund einer Gewichtsabweichung detektiert werden können, während trotzdem die optischen individuellen Schlüsselmerkmale der jeweiligen Instrumente nochmals überprüfbar sind. Dadurch kann sichergestellt werden, dass wirklich jedes der vorgesehenen Instrumente des Instrumentensets im Instrumentensieb 210 angeordnet wurde und korrekt zusammengebaut ist.

Um diese Daten aufnehmen zu können, weist die Datenverarbeitungsanlage 212 neben einer ersten Schnittstelle 226 nun eine zweite Schnittstelle 228 und dritte Schnittstelle 230 auf. Dies ist insbesondere in Fig. 7 zu sehen. Alle Schnittstellen 226, 228 und 230 geben ihre Daten an eine Auswerteeinheit 232 weiter, wie dies durch Pfeile 234, 234' und 234" angedeutet ist. Die Auswerteeinheit 232 kann nun die so erhaltenen Daten mit einer Datenbank 236 austauschen bzw. mit den Referenzdaten aus dieser Datenbank 236 vergleichen. Dies ist schematisch durch Pfeile 238 und 239 angedeutet. Somit können in der Auswerteeinheit 232 neben den Bilddaten aus den jeweiligen Instrumenterfassungseinheiten auch die Bilddaten der Groberkennungseinheit 200 sowie die Wägedaten der Groberkennungseinheit 200 mit entsprechenden Referenzdaten verglichen werden.

Im Übrigen weist die Datenverarbeitungsanlage 212 auch einen Anschluss 240 zur Ausgabe der Daten an eine nicht näher gezeigte Anzeigeeinheit auf. Auch kann bei der Datenverarbeitungsanlage 212 wieder optional eine Steuereinheit 242 vorgesehen sein, um entsprechende Vorrichtungen oder Einheiten aufgrund der ausgewerteten Daten zu steuern. Hierbei ist die Übertragung der Daten von der Auswerteeinheit 232 auf die Steuereinheit 242 durch einen Pfeil 244 angedeutet. Die Ansteuerung von externen Einheiten und Vorrichtungen deutet ein Pfeil 246 an.

Fig. 8 zeigt eine Kameraanordnung 250. Diese Kameraanordnung 250 kann mit ihrer Ausgestaltung auf die jeweiligen Instrumenterfassungseinheiten 12, 102, 152 oder Groberkennungseinheiten 106 und 200 entsprechend übertragen werden.

Die Kameraanordnung 250 weist eine Kamera 252 auf, die ebenfalls oberhalb einer Auflage 254 angeordnet ist. Die Anordnung der Kamera 252 oberhalb der Auflage 254 erfolgt mit Hilfe eines Stativs 256. Die Kameraanordnung 250 ist hierbei so, dass die Kamera 252 in ihrer Position veränderbar angeordnet ist und dabei eine halbkugelförmige Fläche an eigenen Positionen abdecken kann. Dabei ist die Kamera 252 so angeordnet, dass sie stets mit ihrer Kameraperspektive in Richtung auf die Auflage 254 ausgerichtet ist.

In dem vorliegenden Beispiel wird dies dadurch erreicht, dass das Stativ 256 einen Bogen 258 aufweist. An diesem Bogen 258 ist die Kamera 252 beweglich entlang dieses Bogens 258 angeordnet. Diese Bewegung bzw. auch Anordnung kann beispielsweise durch einen hier schematisch dargestellten Motor 260 erfolgen. Der Bogen 258 ist seinerseits an einem Ende über einen zweiten Motor 262 an dem Stativ 256 angeordnet.

Der Bogen 258 kann somit in der Folge Rotationen um eine Achse 264 des Motors 262 durchführen, wie dies durch einen Doppelpfeil 266 angedeutet ist. Ebenso kann die Kamera 252 durch den Motor 260 entlang des Bogens 258 bewegt werden, wie dies durch einen Doppelpfeil 268 angedeutet ist.

Letztendlich gestattet es diese Ausgestaltung einer Kameraanordnung 250 für die jeweiligen Instrument- und Groberkennungseinheiten Bilddaten bzw. Aufnahmen aus zwei oder beliebig vielen unterschiedlichen Perspektiven vornehmen zu können.

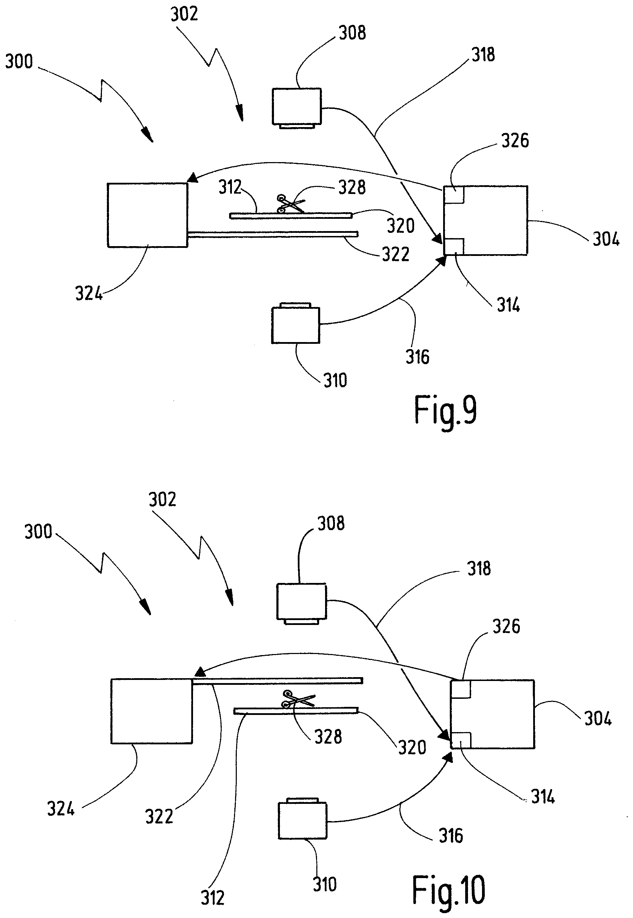

Fig. 9 und 10 zeigen eine weitere erfindungsgemäße Vorrichtung 300. Die Vorrichtung 300 weist eine Instrumenterfassungseinheit 302, eine Datenverarbeitungsanlage 304 und optional eine Groberkennungseinheit auf, wobei die Letztere zum Zwecke der Übersichtlichkeit nicht näher dargestellt ist. Diese Groberkennungseinheit ist so ausgestaltet, wie eine der zuvor bereits gezeigten und erläuterten Groberkennungseinheiten 106, 156 oder 200.

Neben einer ersten Kamera 308 weist die Instrumenterfassungseinheit 302 zusätzlich eine zweite Kamera 310 auf. Die erste Kamera 308 und zweite Kamera 310 sind so zueinander angeordnet, dass sie jeweils auf einer Seite einer Auflage 312 der Instrumenterfassungseinheit 302 angeordnet sind.

Beide Kameras 308 und 310 stehen mit der Datenverarbeitungsanlage 304 über eine hier schematisch angedeutete erste Schnittstelle 314 in Verbindung. Dies ist schematisch durch Pfeile 316 und 318 angedeutet.

Die Auflage 312 weist im vorliegenden Ausführungsbeispiel eine transparente Grundfläche 320 sowie einen kontrastverstärkenden Hintergrund 322 auf.

Die transparente Grundfläche 320 kann dabei aus beliebigen transparenten Materialien gefertigt sein. Diese müssen es lediglich gestatten, entsprechende Bildaufnahmen zu ermöglichen und Instrumente auf diese Grundfläche 320 aufzulegen. Als Beispiele seien hierfür Glas oder transparente Kunststoffe, wie z.B. Plexiglas genannt.

Der kontrastverstärkende Hintergrund 322 ist hier als eine bewegliche einfarbige Platte ausgebildet. Ferner ist der kontrastverstärkende Hintergrund 322 an einer Bewegungseinheit 324 angeordnet. Diese Bewegungseinheit 324 vermag den kontrastverstärkenden Hintergrund 322 von einer Position unterhalb der transparenten Grundfläche 320 in eine Position oberhalb der transparenten Grundfläche 320 zu übertragen - jeweils in Bezug auf die Darstellung der Fig. 9 und 10. Dies kann beispielsweise durch Rotation oder durch eine Bewegungsabfolge der Art seitliches Verschieben, Anheben und wieder Zurückbewegen erfolgen. Ebenfalls denkbar ist eine hier nicht gezeigte Anordnung auf Rollbändern, die neben der transparenten Grundfläche 320 verlaufen und es gestatten, den kontrastverstärkenden Hintergrund 322 zum einen seitlich neben die transparente Grundfläche 320 zu verschieben und vertikal in der Höhe zu verstellen, um ihn dann anschließend wieder über bzw. unter die transparente Grundfläche 320 zurückzuschieben.

Die Bewegungseinheit 324 steht im Übrigen mit einer hier ebenfalls nur schematisch angedeuteten Steuereinheit 326 der Datenverarbeitungsanlage 304 in Verbindung.

Diese Steuereinheit 326 erhält, wie dies bereits in den zuvor beschriebenen Ausführungsbeispielen der Datenverarbeitungsanlagen als Option erläutert wurde, Informationen und Signale von einer hier nicht näher gezeigten Auswerteeinheit der Datenverarbeitungsanlage 304 und regelt somit in diesem Fall das Verschieben und Verstellen des kontrastverstärkenden Hintergrunds 322 über die Bewegungseinheit 324.

Zum Zwecke einer kompletten Automatisierung wäre es ferner denkbar, dass die Steuereinheit 326 hier auch das Auslösen der ersten Kamera 308 und zweiten Kamera 310 und die damit verbundene Erfassung der Bilddaten steuert.

Hinsichtlich des Vorgangs der Erfassung der Bilddaten eines entsprechend hier schematisch angedeuteten Instruments, eine Schere 328, wird zunächst durch die erste Kamera 308 eine Bilderfassung der Schere 328 vorgenommen. Der kontrastverstärkenden Hintergrund 322 ist dabei so unterhalb der transparenten Grundfläche 320 angeordnet, dass dieser weiterhin die Bilderfassungseigenschaften positiv beeinflusst, indem er den Kontrast zwischen dem zu untersuchenden Instrument, hier der Schere 328, und dem Hintergrund erhöht.

Anschließend wird nach Eingang der Bilddaten bei der Datenverarbeitungsanlage 308 mittels der Steuereinheit 328 über die Bewegungseinheit 324 der kontrastverstärkende Hintergrund 322 in seiner Position verschoben. Dabei wird er von unterhalb der transparenten Grundfläche 320 in eine Position oberhalb der transparenten Grundfläche 320 überbracht. Dies ist anhand des Übergangs von Fig. 9 zu Fig. 10 nachvollziehbar.

Nach erfolgter Positionierung des kontrastverstärkenden Hintergrunds 322 kann nun die zweite Kamera 310 mit der Erfassung der Bilddaten beginnen. Hierbei kann diese durch die transparente Grundfläche 320 hindurch die Bilddaten der mit Bezug auf die Darstellung von Fig. 10 unteren Seite der Schere 328 erfassen. Auch hier sorgt wieder der kontrastverstärkende Hintergrund 322 für einen ausreichenden Unterschied zwischen zu untersuchendem Instrument und Hintergrund, um eine Bilderfassung und nachfolgende Untersuchung auf Anomalien zu begünstigen.

Wenngleich die Beschreibung dieses Verfahrens in dieser Reihenfolge vorgenommen wurde, ist es auch möglich, die Reihenfolge umzukehren, d.h. zunächst die Unterseite und dann die Oberseite einer Schere 328 in Form von Bilddaten zu erfassen.

Die so erhaltenen Bilddaten von erster Kamera 308 und zweiter Kamera 310 werden sodann an die Datenverarbeitungsanlage 304 übermittelt und entsprechend den zuvor gemachten Ausführungen in der hier nicht näher gezeigten Auswerteeinheit der Datenverarbeitungsanlage 304 ausgewertet. Dadurch wird das Instrument, hier die Schere 328, untersucht und hinsichtlich jedweder Anomalien analysiert.

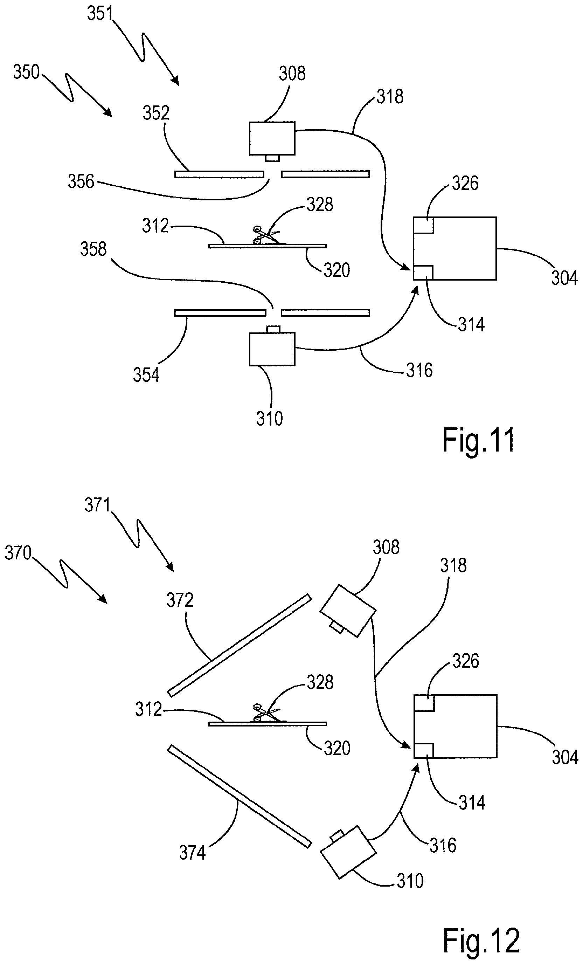

Die in Fig. 11 und 12 dargestellten Vorrichtungen 350 und 370 weisen teilweise gleiche Komponenten wie die Vorrichtung 300 aus Fig. 9 und 10 auf. Entsprechend sind gleiche Merkmale mit den gleichen Bezugszeichen versehen und werden im Folgenden nicht näher im Detail erläutert.

Im Unterschied zur Vorrichtung 300 aus Fig. 9 und 10 weist die Vorrichtung 350 aus Fig. 11 eine Instrumenterfassungseinheit 351 mit zwei kontrastverstärkenden Hintergründen 352 und 354 auf. Diese sind auf jeweils einer Seite der ersten Auflage 312 angeordnet. Dabei befinden sich diese kontrastverstärkenden Hintergründe 352 und 354 jeweils zwischen der ersten Auflage 312 und der jeweiligen Kamera 308 bzw. 310. Um dennoch eine Aufnahme der Instrumente auf der ersten Auflage 312, bzw. der transparenten Grundfläche 320 zu ermöglichen, weisen die kontrastverstärkenden Hintergründe 352 und 354 jeweils eine Öffnung 356 bzw. 358 auf. Durch diese Öffnungen 356 und 358 können die Kameras 308 und 310 jeweils Aufnahmen von einem Instrument, z.B. der Schere 328, auf der transparenten Grundfläche 320 machen. Dabei dient dann jeweils der gegenüberliegende kontrastverstärkende Hintergrund 352 bzw. 354 zur Verbesserung der Bildeigenschaften für die Objekterkennung, wie dies bereits zuvor erläutert wurde.

Mit Bezug auf die Darstellung in Fig. 11 dient der kontrastverstärkende Hintergrund 352 als Hintergrund für die Aufnahme mit Kamera 310 und der kontrastverstärkende Hintergrund 354 als Hintergrund für die Aufnahme mit Kamera 308. Dabei auftretende Aufnahmen der jeweils gegenüberliegenden Kamera 308 bzw. 310 und der entsprechenden Öffnungen 356 bzw. 358 können bei der Bildverarbeitung entsprechend berücksichtigt und aus den Bilddaten herausgerechnet werden. Dies ist beispielsweise durch die Bildung von Differenzbildern, d.h. durch Aufnahmen mit und ohne Instrumenten möglich. Mit der Vorrichtung 350 sind aufgrund der in diesem Beispiel fixen Anordnung von Kamera 308 und 310, sowie kontrastverstärkenden Hintergründen 352 und 354 sogar gleichzeitige und damit zeitsparenden Aufnahmen möglich, da ein Um- bzw. Verstellen von Kamera und/oder kontrastverstärkendem Hintergrund entfällt.

Auch die in Fig. 12 dargestellte Vorrichtung 370 weist eine Instrumenterfassungseinheit 371 mit zwei kontrastverstärkenden Hintergründen 372 und 374 auf. Im Gegensatz zur Vorrichtung 350 aus Fig. 11 sind diese jedoch mit Bezug zu einer durch die erste Auflage 312 bzw. die transparente Grundfläche 320 angedeutete Ebene schräg ausgerichtet. Diese Ausrichtung der kontrastverstärkenden Hintergründe 372 und 374 ist so, dass sie jeweils als Hintergrund für die ebenfalls hier mit ihrer jeweiligen Perspektive schräg zur transparenten Grundfläche 320 angeordneten Kamera 308 bzw. 310 dienen und dadurch entsprechend der zuvor gemachten Ausführungen die Objekterkennung in den Aufnahmen durch die Kameras 308 und 310 verbessern bzw. erleichtern. Die hier im Beispiel gezeigte Anordnung ermöglicht es, dass z.B. die Aufnahme der Schere 328 durch die Kamera 310 vordem kontrastverstärkendem Hintergrund 372 erfolgt. Entsprechend dient der kontrastverstärkende Hintergrund 374 als Hintergrund für Aufnahmen mit der Kamera 308. Die Kamera 308 bzw. 310 ist hier seitlich zum kontrastverstärkenden Hintergrund 372 bzw. 374 angeordnet. Dieser kann daher ohne eine Öffnung, wie sie bei der Vorrichtung 350 in den kontrastverstärkenden Hintergründen 352 und 354 vorliegt, ausgestaltet werden. Eine Aufnahme durch diesen hindurch wird somit durch die jeweils schrägen Anordnungen vermieden. Ebenfalls entfällt ein eventuell notwendiges Bearbeiten der Bilddaten aufgrund der gleichzeitig mit aufgenommenen Kamera 308 bzw. 310 und der jeweiligen Öffnungen 356 bzw. 358.

Auch hierbei wird eine Aufnahme mit beiden Kameras 308 und 310 gleichzeitig und ohne weitere Verzögerungen durch ein Verstellen eines kontrastverstärkenden Hintergrundes und/oder einer Kamera ermöglicht. Kamera 308 und 310 sowie kontrastverstärkende Hintergründe 372 und 374 sind hierfür ebenfalls vorzugsweise fest bzw. fix angeordnet.

Es versteht sich, dass die zuvor gemachten Ausführungen zur Ausgestaltung einer Kamera, insbesondere in Fig. 8, auch auf die hier gezeigten Kameras 308 und 310 der Fig. 9, 10, 11 und 12 übertragbar sind. Ferner versteht sich auch, dass entsprechende Beleuchtungseinheiten in dieser Vorrichtung vorgesehen sind und lediglich zum Zwecke der Vereinfachung und Übersichtlichkeit der Darstellungen nicht näher gezeigt sind. Neben der zuvor dargestellten Verwendung von Lampen ist es im Übrigen auch denkbar, in den hier gezeigten Kameras 308 und 310, sowie auch in allen anderen im vorhergehenden beschriebenen Kameras und Kameravorrichtungen, Beleuchtungseinrichtungen in oder an der Kamera selber anzuordnen. Dadurch ergibt sich eine kompakte platzsparende Anordnung.

Ferner versteht sich, dass auch die einzeln dargestellten Instrumenterfassungseinheiten 302, 351 und 371 jeweils optional mit den zuvor beschriebenen Groberkennungseinheiten 106, 156 bzw. 200 kombiniert werden können. Vorzugsweise weisen die Vorrichtungen 350 und 370 ebenfalls eine Groberkennungseinheit auf, die jeweils entsprechend einer der Groberkennungseinheiten 106, 156 oder 200 ausgestaltet ist.

Neben der zuvor beschriebenen Verwendung der Instrumenterfassungseinheiten 12, 102, 152, 302, 351 und 371 zum Zwecke der Untersuchung der auf die jeweiligen Auflagen aufgelegten Instrumente auf Anomalien, kann in einer bevorzugten Ausgestaltung der vorliegenden Erfindung auch vorgesehen sein, dass die jeweiligen Datenverarbeitungsanlagen 14, 104, 154, 212 und 314 so ausgestaltet sind, dass sie ferner eine Erkennung der aufgelegten Instrumente ermöglichen. Diese Erkennung der Instrumente kann beispielsweise mit Hilfe von speziell abgestimmten Objekterkennungsalgorithmen, wie z.B. Korrelationsmethoden und Methoden der kantenbasierten Objekterkennung (z.B. generalisierte Hough-Transformation), die einzelnen Objekte, d.h. in diesem Fall die aufgelegten Instrumente, anhand der erfassten Bilddaten erkennen.

Dazu können in den jeweiligen Datenbanken dann entsprechende Referenzbilder hinterlegt sein, mit denen die jeweiligen Auswerteeinheiten die erkannten Instrumente vergleichen können.

Die erkannten und identifizierten Instrumente werden sodann mit ebenfalls vorzugsweise in der Datenverarbeitungsanlage hinterlegten Packlisten verglichen, auf denen die Instrumente aufgeführt sind, die zu einem zu packenden Instrumentenset gehören.

So ist auf vorteilhafte Weise möglich, zusätzlich zu einer auf Objekterkennung basierten Abarbeitung von Packlisten zum Bestücken von Instrumentensets gleichzeitig eine Untersuchung der aufgelegten Instrumente auf jedwede Art von Anomalien entsprechend der vorliegenden Erfindung durchzuführen.

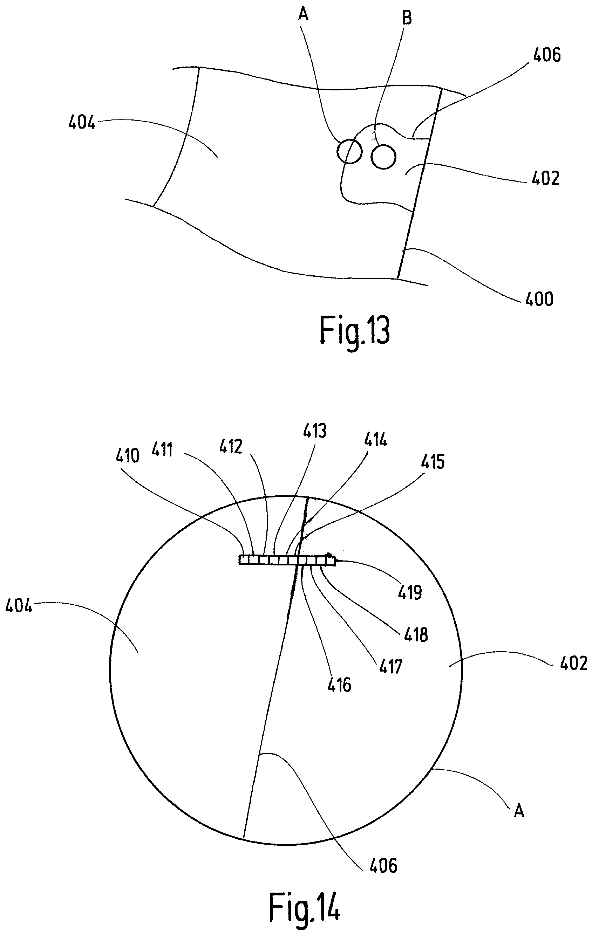

Die zuvor erwähnte Untersuchung der Instrumente auf Anomalien soll im Folgenden nun exemplarisch anhand der Fig. 13 bis 15 hinsichtlich der erfindungsgemäßen Vorrichtung und des Verfahrens erläutert werden.

Der in der Fig. 13 dargestellte Ausschnitt eines Instruments 400 kann beispielsweise der Ausschnitt des Griffs der zuvor erwähnten Scheren 58 oder 328 oder der Klemme 64 sein.

Dieses Instrument 400 weist in dem hier angedeuteten Ausschnitt eine Anomalie 402 neben einem unbeschadeten Bereich 404 auf. Diese Anomalie 402 kann rein theoretisch erst einmal ein regulärer Bestandteil des Instruments, wie z.B. ein Griff, eine Hülse, eine farbig andersartige Niete oder dgl., sein. Daneben kann es sich aber auch, wie es für dieses Beispiel angenommen wird, um einen schadhaften Bereich handeln. Als schadhafte Bereiche kommen u.a. Korrosion, wie Spannungs-, Flächen-, Kontakt-, Reibungskorrosionen oder dgl., Verunreinigung, z.B. durch organische Rückstände, oder sonstige Verfärbungen in Frage. Für die folgenden Erläuterungen wird hierbei exemplarisch angenommen, dass es sich bei der Anomalie 402 um eine Korrosion handelt.

Ferner wird für die folgenden Erläuterungen angenommen, dass die Anomalie 402 und der unbeschadete Bereich 404 durch einen eindeutigen Übergang 406 voneinander getrennt sind. Solch ein Übergang 406 ist für die Funktionsweise der erfindungsgemäßen Vorrichtungen und des Verfahrens nicht notwendig und wird in der Realität oftmals auch nicht zu finden sein. Er dient im Vorliegenden lediglich dazu, die Erfindung anhand des Beispiels der Fig. 13 möglichst übersichtlich und einfach zu veranschaulichen.

Um das Instrument 400 auf eventuelle Anomalien zu untersuchen, wird zunächst das Instrument auf eine Auflage, beispielsweise die Auflage 16 der Vorrichtung 10 gelegt und mit Hilfe der ersten Kamera 18 die Bilddaten dieses Instruments 400 erfasst. Diese Bilddaten werden dann von der Auswerteeinheit 26 auf Regionen untersucht, die Anomalien aufweisen.

Dabei wird im Rahmen dieser Untersuchung und Analyse der Bilddaten des Instruments zunächst nach farblich auffälligen Regionen gesucht. Dieses Suchen nach farblich auffälligen Regionen verläuft im vorliegenden Ausführungsbeispiel so, dass die Farbstärke eines jeden Pixels der erhaltenen Bilddaten bestimmt wird. Dabei wird zuallererst mit Hilfe des kontrastverstärkenden Hintergrunds 66 der Hintergrund aus den Bilddaten herausgefiltert, um lediglich die reinen Bilddaten der zu untersuchenden Instrumente bzw. des zu untersuchenden Instruments 400 vorliegen zu haben.

Die Bestimmung der Farbstärke verläuft dann im vorliegenden exemplarischen additiven RGB-Farbraum so, dass die jeweiligen Unterschiede zwischen den einzelnen Bildkanälen bestimmt werden. Dies kann z.B. mit der folgenden Formel 1 geschehen:

Formel 1:

$$FS(x,y) = \frac{\left|BK_1(x,y) - BK_2(x,y)\right| + \left|BK_2(x,y) - BK_3(x,y)\right| + \left|BK_3(x,y) - BK_1(x,y)\right|}{3}$$

Diese Formel bezieht sich auf die Farbstärke für ein einzelnes Pixel mit den Koordinaten (x,y) einer Rastergraphik. Dabei bezeichnet FS die Farbstärke und $BK_i(x,y)$ den jeweiligen Bildkanalwert in einen der drei Bildkanäle (mit i = 1, 2 oder 3) des Pixels an der Stelle (x,y) im RGB-Farbraum. Im Rahmen des vorliegenden Beispiels repräsentiert jeder Bildkanal einen Kanal der RGB-Daten, d.h. eine der Farben Rot, Grün oder Blau. Bezogen auf die vorliegenden medizinischen Instrumente würde dann eine häufig vorkommende unbeschadete metallische Oberfläche in allen drei verfügbaren Bildkanälen sehr hohe Werte aufweisen. Dies würde entsprechend der Formel 1 dazu führen, dass die einzelnen Terme zwischen den Betragsstrichen durch die nahezu identischen Werte in den einzelnen Bildkanälen sehr gering oder null werden. Das Ergebnis ist ein geringer Wert für die Farbstärke FS(x,y).

Als andere Alternative kann auch ein häufig vorkommender schwarzer Kunststoffbestandteil des medizinischen Instruments vorliegen, der dann in jedem einzelnen Bildkanal sehr niedrige Werte aufweisen würde. Auch hierbei würde die Ähnlichkeit der einzelnen Werte untereinander dazu führen, dass die einzelnen Terme in den Betragsstrichen wiederum sehr gering oder null werden und folglich auch der Wert für die Farbstärke FS(x,y) sehr gering wird.

Liegt nun der Fall einer Anomalie, wie z.B. die Anomalie 402 in Form einer Korrosion vor, kommt es zu unterschiedlichen Bildkanalwerten in den einzelnen Bildkanälen. Hierzu soll angenommen werden, dass eine entsprechende Korrosion farblich so ausgestaltet ist, dass sie einen deutlichen Rotanteil, jedoch geringere Grün- und Blauanteile aufweist. In Bezug auf die Bildkanäle des RGB-Farbraums bedeutet dies dann, dass in diesem Beispiel ein Bildkanal einen deutlich höheren Wert aufweist als die anderen beiden Bildkanäle. Mit Bezug auf Formel 1 führt dies dann dazu, dass bei weitestgehender Ähnlichkeit der anderen beiden Bildkanäle der Differenz-bildende Term dieser beiden Bildkanäle gering bzw. nahezu null wird. Demgegenüber nehmen die anderen beiden verbleibenden Terme, die den Bildkanal mit dem hohen Wert aufweisen und im Vergleich zu den verbleibenden beiden Bildkanälen stehen, relativ große Werte an. Dies führt im Ergebnis der Formel 1 dann zu einem vergleichsweise hohen Wert der Farbstärke FS.

Um geringe farbliche Abweichungen in Form von Unterschieden zwischen den Bildkanälen einerseits und tatsächlich farblich auffällige Regionen andererseits, insbesondere Anomalien, zu unterscheiden, wird vorzugsweise in dem erfindungsgemäßen Verfahren und bei der erfindungsgemäßen Vorrichtung ein Schwellwert vorgesehen, den die Farbstärke FS nach Formel 1 überschreiten muss. Nur so wird die farbliche Abweichung als Anomalie, also farblich auffällige Region, eingeordnet. Dieser Schwellwert wird in Abhängigkeit der verwendeten medizinischen Instrumente empirisch, d.h. durch Versuche mit einer Reihe von medizinischen Instrumenten des verwendeten Bestands ermittelt und festgelegt.

Hierbei sei anzumerken, dass dieser Schwellwert für den gesamten Betrieb der Vorrichtung und des erfindungsgemäßen Verfahrens nicht auf Dauer fix angelegt sein muss. Dieser kann sich auch im Laufe des Betriebs und der Anwendung des Verfahrens aufgrund von Anpassungen durch den Benutzer ändern. Dies ist z.B. dann angebracht, wenn neue Instrumente dem Bestand hinzugefügt werden, die mit der erfindungsgemäßen Vorrichtung und dem Verfahren untersucht werden sollen. Auch kommt dies in Frage, wenn die verwendeten Instrumente an sich einer leichten Grundverfärbung unterliegen und somit eine Anpassung des Schwellwerts notwendig wird. Ebenfalls kommt dies auch in Betracht, wenn aus irgendeinem anderen beliebigen Grund die Sensitivität der Vorrichtung bzw. des Verfahrens verstärkt (Verringerung des Schwellwerts) oder verringert (Erhöhung des Schwellwerts) werden soll.

Die Unterscheidung zwischen dem unbeschadeten Bereich 404 und der Anomalie 402 soll im Folgenden nun näher anhand der vergrößerten Darstellung von Fig. 14 beschrieben werden. Hierbei ist in diesem stark vergrößerten Ausschnitt des Instruments 400 der Übergangsbereich vom unbeschadeten Bereich 404 in die Anomalie 402 mit dem Übergang 406 zu sehen. Dabei sind schematisch einzelne Pixel 410 bis 419 angedeutet. Von diesen fallen die Pixel 410 bis 415 in den unbeschadeten Bereich 404, während die Pixel 416 bis 419 in den Bereich der Anomalie 402 fallen. Es versteht sich, dass die Bilddaten insgesamt als eine Ansammlung von Pixeln vorliegen, so dass die hier gezeigte Reihe der zehn Pixel 410 bis 419 lediglich zur Veranschaulichung der Funktionsweise der Vorrichtung und des Verfahrens dient und auf die Darstellung aller Pixel, die letztendlich die gesamten Darstellungen ausfüllen würden, zum Zwecke der Übersichtlichkeit verzichtet wurde.

Im Rahmen der vorliegenden Erfindung würde dann jeweils die Farbstärke der hier gezeigten Pixel im nächsten Schritt untersucht werden.

Dies würde im Ergebnis dazu führen, dass für alle Pixel 410 bis 415 ein vergleichsweise geringer Wert für die Farbstärke FS ermittelt wird. Dies liegt entsprechend der zuvor gemachten Ausführung daran, dass der unbeschadete Bereich 404 im vorliegenden Beispiel metallisch ist, so dass für alle Bildkanäle recht hohe Werte vorliegen. Dies führt entsprechend der Formel 1 dazu, dass der Gesamtwert FS gering ausfällt. Die Farbstärken der Pixel 410 bis 415 des unbeschadeten Bereichs 404 liegen somit auch unter dem Schwellwert. Entsprechend würde im Rahmen der vorliegenden Erfindung dieser unbeschadete Bereich 404 auch als solch ein Bereich ohne farblich auffällige Region und somit ohne Fehler oder Schäden erkannt werden.

Beim weiteren Durchgang der Pixel würde dann bei den nachfolgenden hier exemplarisch dargestellten Pixeln 416 bis 419 ein anderes Ergebnis für die Farbstärke FS erhalten werden. Anhand des zuvor aufgeführten Beispiels der Korrosion wäre es hier so, dass in einem Bildkanal recht hohe Werte zu erwarten sind, während die anderen Bildkanäle vergleichsweise geringe Werte aufweisen würden. Mit Bezug auf die zuvor gemachten Ausführungen führt dies dann dazu, dass für die Farbstärke FS gemäß Formel 1 ein hoher Wert erreicht wird, der über dem Schwellwert liegt. Somit erfolgt für jedes der Pixel 416 bis 419 eine Einstufung, die als farblich auffällig bezeichnet werden kann.

Anschließend werden dann erfindungsgemäß alle im Bereich der Anomalie 402 liegenden Pixel zu einer Gruppe zusammengefasst. Diese Gruppe besteht dann aus einander benachbarten farblich auffälligen Pixeln. Ferner beschreibt diese Gruppe dann eine farblich auffällige Region, nämlich die Anomalie 402.

Nach Auffinden aller der zur Anomalie 402 zugehörigen Pixel wird dann dem Benutzer mitgeteilt, dass das untersuchte Instrument 400 eine Anomalie 402 aufweist. Ferner ist auch denkbar, dass die erfindungsgemäße Vorrichtung weitere Einrichtungen, wie z.B. Roboter, aufweist, die dann ein solches Instrument mit einer Anomalie 402 aussortieren. Solche Einrichtungen sind hier jedoch nicht näher gezeigt.

In einer bevorzugten Ausgestaltung der Erfindung wird bei der Vorrichtung und dem Verfahren dann dahingehend fortgefahren, dass die aufgefundene Anomalie 402 ferner identifiziert wird.

Dazu wird diese farblich auffällige Region in Form der Anomalie 402 klassifiziert. Hierzu ist in der Datenverarbeitungsanlage 14, insbesondere der Datenbank 28, hinterlegt, welche unterschiedlichen Klassen von farblich auffälligen Regionen, also Anomalien, auftreten können.

Diese Hinterlegung in der Datenverarbeitungsanlage 14 kann z.B. vom Hersteller der Vorrichtung bereits vorgegeben sein. Vorzugsweise wird diese Ansammlung von Klassen jedoch mit dem System in Form der Vorrichtung und des Verfahrens erlernt. Dazu wird eine Reihe von vorkommenden Instrumenten nacheinander durch das System untersucht und alle als Anomalien erkannten farblich auffälligen Regionen dem Benutzer angezeigt. Dieser untersucht im Rahmen dieser Lernphase dann seinerseits die Instrumente an diesen erkannten Anomalien und ordnet sie bestimmten Klassen zu. Dies kann beispielsweise durch Eingabe über die Tastatur 54 geschehen.

Die so erzeugten bzw. eingegebenen und zugeordneten Klassen können sowohl Schäden und Verunreinigungen als auch farblich andersartige Instrumententeile, wie z.B. farbige Griffe, umfassen.

Für jede bestimmte Klasse ergibt sich dann anhand der in den Anomalien vorkommenden Pixeln mit ihren unterschiedlichen Farbinformationen eine Farbverteilung. Diese liegt im hier exemplarisch besprochenen dreidimensionalen RGB-Farbraum als 3D-Histogramm vor.

Dieses 3D-Histogramm weist in der Regel teilweise ungleichmäßige Verläufe auf, die optional durch Faltung mit einer 3D-Gauß-Funktion zu glatteren Wahrscheinlichkeitsfunktionen für die klassenspezifischen Farbverteilungen überarbeitet werden können.

Letztendlich kann aus dem so erhaltenen 3D-Histogramm dann für jede Farbinformation in Form der drei Bildkanäle eine Wahrscheinlichkeit bestimmt werden. Da jedes 3D-Histogramm für jede eingegebene Klasse unterschiedlich ist, ergeben sich auch für eine aus einem Bildpixel erkannte bzw. resultierende Farbinformation für jede dieser Klassen unterschiedliche bedingte Wahrscheinlichkeiten, dass eine solche Farbinformation zu einer entsprechenden Klasse gehört bzw. zugeordnet werden kann.

Diese zuvor genannte Farbinformation kann dabei als Transformation $\vec{F}$ des Pixels (x,y) in den RGB-Farbraum angesehen werden:

Formel 2:

$$\vec{F}(x, y) = (r, g, b)$$

Basierend auf der zuvor dargestellten Lernphase kann eine sog. a priori-Wahrscheinlichkeit $Pr(C_i)$ ermittelt werden, die die Wahrscheinlichkeit darstellt, dass eine entsprechende Klasse $C_i$ unter statistischen Gesichtspunkten in Relation zu allen vorhandenen Klassen auftritt.

Insgesamt wird dann die Bestimmung der Wahrscheinlichkeit, dass ein Pixel zu einer bestimmten Klasse zugehört, anhand der nachfolgend in Formel 3 dargestellten a posteriori-Wahrscheinlichkeit bestimmt werden:

Formel 3:

$$a\_Posteriori_i\left(C_i\middle|\vec{F}\right)=\frac{P_i\left(\vec{F}\middle|C_i\right)\cdot\Pr(C_i)}{\sum_{i=1}^{N}P_i\left(\vec{F}\middle|C_i\right)\cdot\Pr(C_i)}$$

Hierbei ist $C_i$ eine Klasse aus der Menge aller Klassen C, die in der Datenbank hinterlegt sind, $\vec{F}$ die Transformation des Pixels x,y in den RGB-Farbraum, auch Farbwert (oder zuvor Farbinformation) genannt, und $\Pr(C_i)$ die zuvor beschriebene a priori-Wahrscheinlichkeit des Auftretens der Klasse $C_i$ an sich. Der Ausdruck $P_i(\vec{F}|C_i)$ ist die klassenbedingte Wahrscheinlichkeit ("likelihood"). Diese gibt an mit welcher Wahrscheinlichkeit ein Farbwert bzw. eine Farbinformation speziell in der Klasse $C_i$ auftritt. Hierzu kann anhand der klassenbezogenen Sammlung von Farbwerten $\vec{F}$ während einer Trainingsphase für jede Klasse $C_i$ ein (geglättetes) Histogramm bestimmt werden. Dieses Histogramm gibt dann an, mit welcher Wahrscheinlichkeit ein Farbwert $\vec{F}$ zu einer speziellen Klasse $C_i$ gehört.

Diese Formel 3 ist in ihrer allgemeinen Form auch als Bayes-Formel bekannt und basiert auf dem Bayes-Theorem.

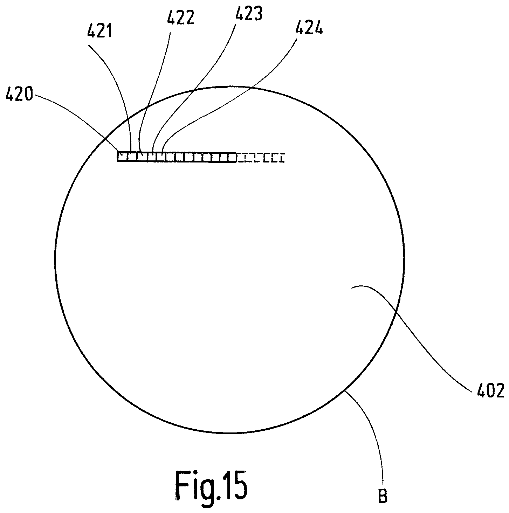

In der Darstellung der Fig. 15 sind exemplarisch einige Bildpixel, hier nun ausschließlich der Anomalie 402, dargestellt, wobei hier ausschließlich die Pixel 420 bis 424 mit Bezugszeichen versehen sind.

Entsprechend der zuvor dargestellten Formel 3 zur Bestimmung der a posteriori-Wahrscheinlichkeit, dass ein Pixel basierend auf der aus ihm erhaltenen Farbinformation zu einer bestimmten Klasse $C_i$ zugehört, wird für jedes der Pixel 420 bis 424 jeweils die entsprechende a posteriori-Wahrscheinlichkeit bezüglich jeder im System der Datenverarbeitungsanlage 14 vorliegenden Klasse ermittelt.

Um nun eine Zuordnung eines Pixels zu einer Klasse zu treffen, wird bei jedem Pixel 420 bis 424 jeweils die größte Wahrscheinlichkeit, also das Maximum der a posteriori-Wahrscheinlichkeiten, über alle Klassen ermittelt. Dies ist durch die folgende Formel 4 veranschaulicht:

Formel 4:

$$c_{MAP}\left(\vec{F}(x,y)\right)=\arg\max_{c\in C} a\_Posteriori\left(c\middle|\vec{F}(x,y)\right)$$

Hierbei ist c eine Klasse aus der Menge aller Klassen C. MAP bedeutet Maximum A Posteriori. $c_{MAP}$ ist somit die Klasse mit der größten a posteriori-Wahrscheinlichkeit, die dann dem jeweiligen Pixel zugeordnet wird.

Auf die gesamte Anomalie 402 bezogen, bedeutet dies also, dass für alle gezeigten Pixel 420 bis 424 und für alle hier nicht näher bezeichneten und gezeigten verbleibenden Pixel der Anomalie 402 jeweils die Klasse mit der größten Wahrscheinlichkeit $c_{MAP}$ ermittelt wird. Da es nun aber vorkommen kann, dass z.B. für das Pixel 420 die Klasse $C_1$ und für das Pixel 421 die Klasse $C_2$ ermittelt wird, wird auch unter allen Pixeln der Anomalie 402, ein Maximum ermittelt werden.

Dies geschieht durch Bestimmung der am häufigsten auftretenden Klasse aus der Menge aller vorhandenen Pixel der Anomalie 402. Dies ist durch die nachfolgende Formel 5 veranschaulicht:

Formel 5:

$$c_{Anomalie}=\arg\max_{c\in C}\sum_{j=1}^{M_{Anomalie}}c_{MAP}\left(\vec{F}(x,y)_j\right)$$

Dabei ist $c_{Anomalie}$ die wahrscheinlichste Zuordnung der entdeckten Anomalie 402 zu einer Klasse und $M_{Anomalie}$ die Anzahl aller Pixel in der untersuchten Anomalie, hier der Anomalie 402.

Nach Identifizieren und Zuordnen dieser Klasse $c_{Anomalie}$ zu der aufgefundenen farblich auffälligen Region in Form der Anomalie 402, wird dem Benutzer mitgeteilt, um welche Klasse, d.h. um welche Art von Anomalie, es sich handelt. Dies kann zum einen durch die Anzeigeeinheit 38 geschehen.

Alternativ ist auch denkbar, dass hier ebenso weitere Einrichtungen als Bestandteil der erfindungsgemäßen Vorrichtung vorhanden sind, die dann ein entsprechendes Instrument 400, welches eine nun mit $c_{Anomalie}$ klassifizierte Anomalie 402 aufweist, entsprechend weiterbehandeln. Dieses Behandeln kann zum einen das Zuführen zu weiteren Reinigungs- und Sterilisierungsschritten sein, wenn es sich z.B. bei der Anomalie 402 um organische Rückstände oder allgemein Verunreinigungen handelt. Die Klasse $c_{Anomalie}$ wäre dann eine Klasse aus dem Bereich der Verunreinigungen.

Alternativ kann das Instrument 400 auch der Reparatur oder Aussonderung aus dem Bestand zugeführt werden, wenn die erkannte Anomalie 402 eine Beschädigung, z.B. eine Korrosion, ist. Die Klasse $c_{Anomalie}$ würde dann den Schadenklassen zugehören.

Eine solche Einrichtung zur weiteren Behandlung dieser Instrumente kann beispielsweise ein Roboter sein. Dieser Roboter könnte im Übrigen dann im Rahmen dieser Erfindung auch durch die Datenverarbeitungsanlage 14 angesteuert werden. Hierzu kommt die zuvor als Option erwähnte Steuereinheit 44 in Betracht.

Wenngleich im Vorhergehenden das erfindungsgemäße Verfahren und die Vorrichtung im Hinblick auf die Erkennung von Anomalien und farblich auffälligen Regionen im Zusammenhang mit der Vorrichtung 10 beschrieben wurde, versteht sich, dass die zuvor gemachten Ausführungen auch auf die übrigen zuvor erwähnten Vorrichtungen 100, 150, 300, 350 und 370 analog übertragbar sind.

In diesem Zusammenhang ist anzumerken, dass insbesondere eine Ausgestaltung entsprechend der Vorrichtungen 300, 350 und 370 bevorzugt ist, die es gestattet, Bilddaten von Ober- und Unterseite aus zumindest jeweils einer Perspektive, vorzugsweise sogar aus mehreren Perspektiven bei Kombination mit der Kameraanordnung 250 zu erfassen. Dies liegt insbesondere daran, dass hierdurch das komplette Instrument 400 auf Anomalien untersucht werden kann, ohne dass es des Eingriffs eines Benutzers zum Drehen oder Verschieben des Instruments 400 bedarf.

**Patentansprüche**

1. Vorrichtung zum Erkennen von Anomalien an medizinischen Instrumenten, mit einer Datenverarbeitungsanlage (14, 104, 154, 212, 304) und einer Instrumenterfassungseinheit (12, 102, 152, 302, 351, 371), wobei die Datenverarbeitungsanlage (14, 104, 154, 212, 304) eine Anzeigeeinheit (38, 120, 170, 218), eine Datenbank (28, 184, 236), eine erste Schnittstelle (24, 180, 226) und eine Auswerteeinheit (26, 182, 232) aufweist und die Instrumenterfassungseinheit (12, 102, 152, 302, 351, 371) eine Auflage (16, 108, 158, 312) und zumindest eine Kamera (18, 110, 160, 252, 308, 310) aufweist, wobei die zumindest eine Kamera (18, 110, 160, 252, 308, 310) so angeordnet und ausgerichtet ist, dass sie Bilddaten von auf der Auflage (16, 108, 158, 312) angeordneten medizinischen Instrumenten (58, 64, 328, 400) aus zumindest einer Perspektive erfassen kann, und die Datenverarbeitungsanlage (14, 104, 154, 212, 304) so ausgestaltet ist, dass sie über die erste Schnittstelle (24, 180, 226) Bilddaten von der zumindest einen Kamera (18, 110, 160, 252, 308) erhält und die erhaltenen Bilddaten in der Datenbank (28, 184, 236) speichern und mittels der Auswerteeinheit (26, 182, 232) auf Regionen untersuchen kann, die Anomalien (402) aufweisen, **dadurch gekennzeichnet, dass** die Datenverarbeitungsanlage (14, 104, 154, 212, 304) ferner so ausgestaltet ist, dass sie die Regionen klassifizieren kann und anhand der Klassifikation bestimmen kann, ob die Regionen jeweils ein regulärer Bestandteil des Instruments (58, 64, 328, 400) oder eine Anomalie (402) sind, und ausgestaltet ist zum Durchführen der folgenden Schritte:

   aa) Auffinden von farblich auffälligen Regionen,
   bb) Klassifizieren der farblich auffälligen Regionen,
   cc) Bestimmen anhand der Klassifikation, ob die farblich auffällige Region ein regulärer Bestandteil des Instruments (58, 64, 328, 400) oder eine Anomalie (402) ist, und
   dd) Bestimmen der Art der Anomalie (402) der farblich auffälligen Region.

2. Vorrichtung nach Anspruch 1, **dadurch gekennzeichnet, dass** die Anomalien (402) Schäden oder Verunreinigungen sind.

3. Vorrichtung nach Anspruch 1 oder 2, **dadurch gekennzeichnet, dass** die Auflage (16, 108, 158, 312) einen kontrastverstärkenden Hintergrund (66, 322, 352, 354, 372, 374) aufweist.

4. Vorrichtung nach einem der Ansprüche 1 bis 3, **dadurch gekennzeichnet, dass** die Kamera (18, 110, 160, 252, 308, 310) in ihrer Position veränderbar angeordnet ist, so dass sie Bilddaten aus zumindest zwei Perspektiven erfassen kann.

5. Vorrichtung nach Anspruch 3 oder 4, **dadurch gekennzeichnet, dass** die Auflage (16, 108, 158, 312) eine transparente Grundfläche (320) aufweist und dass der kontrastverstärkende Hintergrund (62, 322) in seiner Position zwischen den beiden Seiten der transparenten Grundfläche (320) veränderbar angeordnet ist.

6. Vorrichtung nach Anspruch 3 oder 4, **dadurch gekennzeichnet, dass** die Auflage (16, 108, 158, 312) eine transparente Grundfläche (320) und die Vorrichtung zumindest zwei kontrastverstärkende Hintergründe (352, 354, 372, 374) aufweist, die jeweils so auf den gegenüberliegenden Seiten der transparenten Grundfläche (320) angeordnet sind, dass jeweils zumindest ein kontrastverstärkender Hintergrund (352, 354, 372, 374) aus einer jeweiligen Kameraperspektive hinter dem zu erkennenden Instrument (58, 64, 328) liegt

7. Verfahren zum Erkennen von Anomalien an medizinischen Instrumenten mit einer Vorrichtung nach einem der Ansprüche 1 bis 6, das folgende Schritte aufweist:

a) Auflegen von zumindest einem Instrument (58, 64, 328, 400) auf die Auflage der Instrumenterfassungseinheit (12, 102, 152, 302, 351, 371),
b) Erfassen von Bilddaten durch die zumindest eine Kamera (18, 110, 160, 252, 308, 310),
c) Weitergabe der Bilddaten an die Datenverarbeitungsanlage (14, 104, 154, 212, 304),
d) Analyse der Bilddaten auf Anomalien (402) auf dem Instrument (58, 64, 328, 400) aufweisend die Schritte:

aa) Auffinden von farblich auffälligen Regionen,
bb) Klassifizieren der farblich auffälligen Regionen,
cc) Bestimmen anhand der Klassifikation, ob die farblich auffällige Region ein regulärer Bestandteil des Instruments (58, 64, 328, 400) oder eine Anomalie (402) ist, und
dd) Bestimmen der Art der Anomalie (402) der farblich auffälligen Region.

e) Mitteilen der Informationen über die Anomalien (402) an einen Benutzer.

8. Verfahren nach Anspruch 7, **dadurch gekennzeichnet, dass** der Schritt aa) folgende Schritte aufweist:

- Bestimmen der Farbstärke eines jeden Pixels (410-419, 420-424),
- Vergleich der Farbstärke mit einem vorgegebenen Schwellenwert,
- Einstufen des Pixels (410-419, 420-424) als farblich auffällig bei Überschreiten des Schwellenwerts, und
- Zusammenfassen aller einander benachbarten farblich auffälligen Pixel (416-419; 420-424) zu einer farblich auffälligen Region.

9. Verfahren nach Anspruch 8, **dadurch gekennzeichnet, dass** das Bestimmen der Farbstärke durch Ermittlung der Unterschiedlichkeit der Bildkanalwerte der einzelnen Bildkanäle realisiert wird.

10. Verfahren nach einem der Ansprüche 7 bis 9, **dadurch gekennzeichnet, dass** der Schritt bb) folgende Schritte aufweist:

- Ermitteln der Wahrscheinlichkeit für jedes Pixel (410-419; 420-424) einer farblich auffälligen Region zu einer bestimmten Klasse von Anomalien (402) zu gehören,
- Auswählen und Zuweisen der Klasse mit der größten Wahrscheinlichkeit zu dem jeweiligen Pixel (410-419; 420-424), und
- Auswählen der am häufigsten auftretenden Klasse bei der Gesamtheit aller Pixel (410-419; 420-424) der farblich auffälligen Region und Zuweisen dieser Klasse zu der farblich auffälligen Region.

11. Computer-lesbares nicht vorübergehendes Medium auf dem ein Computerprogramm gespeichert ist, das Programmcodebestandteile aufweist, die auf einem Computer ausgeführt dazu führen, dass der Computer die Schritte des Verfahrens nach einem der Ansprüche 7 bis 10 durchführt.

**Claims**

1. Apparatus for identifying anomalies on medical instruments, having a data processing installation (14, 104, 154, 212, 304) and an instrument detection unit (12, 102, 152, 302, 351, 371), wherein the data processing installation (14, 104, 154, 212, 304) has a display unit (38, 120, 170, 218), a database (28, 184, 236), a first interface (24, 180, 226) and an evaluation unit (26, 182, 232) and the instrument detection unit (12, 102, 152, 302, 351, 371) has a support (16, 108, 158, 312) and at least one camera (18, 110, 160, 252, 308, 310), wherein the at least one camera (18, 110, 160, 252, 308, 310) is arranged and oriented such that it can capture image data from medical instruments (58, 64, 328, 400) arranged on the support (16, 108, 158, 312) from at least one perspective, and the data processing installation (14, 104, 154, 212, 304) is adapted such that it uses the first interface (24, 180, 226) to receive image data from the at least one camera (18, 110, 160, 252, 308) and can store the received image data in the database (28, 184, 236) and can use the evaluation unit (26, 182, 232) to examine said image data for regions which have anomalies (402), **characterized in that** the data processing installation (14, 104, 154, 212, 304) is also adapted such that it can classify the regions and can use the classification to determine whether the regions are respectively a regular part of the instrument (58, 64, 328, 400) or an anomaly (402), and adapted to perform the following steps:

aa) finding regions of conspicuous color,
bb) classifying the regions of conspicuous color,
cc) determining, based on the classification, whether the region of conspicuous color is a regular part of the instrument (58, 64, 328, 400) or an anomaly (402), and
dd) determining the type of anomaly (402) of the region of conspicuous color.

2. Apparatus according to claim 1, **characterized in that** the anomalies (402) are damage or soiling.

3. Apparatus according to claim 1 or 2, **characterized in that** the support (16, 108, 158, 312) has a contrast enhancing background (66, 322, 352, 354, 372, 374).

4. Apparatus according to any of claims 1 to 3, **characterized in that** the camera (18, 110, 160, 252, 308, 310) is arranged such that its position can be altered, as a result of which it can capture image data from at least two perspectives.

5. Apparatus according to claim 3 or 4, **characterized in that** the support (16, 108, 158, 312) has a transparent base area (320) and **in that** the contrast-enhancing background (62, 322) is arranged such that its position can be altered between the two sides of the transparent base area (320).

6. Apparatus according to claim 3 or 4, **characterized in that** the support (16, 108, 158, 312) has a transparent base area (320) and **in that** the apparatus has al least two contrast-enhancing backgrounds (352, 354, 372, 374) that are arranged at respectively opposite sides of the transparent base area (320), so that respectively at least one contrast-enhancing background (352, 354, 372, 374) from the respective camera perspective lies behind the instrument (58, 64, 328) to be viewed.

7. Method for identifying anomalies on medical instruments using an apparatus according to any of claims 1 to 6, comprising the following steps:

a) placing at least one instrument (58, 64, 328, 400) onto the support of the instrument detection unit (12, 102, 152, 302),
b) capturing image data by the at least one camera (18, 110, 160, 252, 308, 310),
c) forwarding the image data to the data processing installation (14, 104, 154, 212, 304),
d) analyzing the image data for anomalies (402) on the instrument (58, 64, 328, 400), comprising the steps of:

aa) finding regions of conspicuous color,
bb) classifying the regions of conspicuous color,
cc) determining, based on the classification, whether the region of conspicuous color is a regular part of the instrument (58, 64, 328, 400) or an anomaly (402), and
dd) determining the type of anomaly (402) of the region of conspicuous color,

e) communicating the information about the anomalies (402) to the user.

8. Method according to claim 7, **characterized in that** step aa) comprises the following steps:

- determining the color intensity of each pixel (410-419, 420 424),
- comparing the color intensity with a preset threshold value,
- grading the pixel (410-419, 420 424) as being of conspicuous color if the threshold value is exceeded,
- combining all adjacent pixels of conspicuous color (416-419; 420-424) to form a region of conspicuous color.

9. Method according to claim 8, **characterized in that** determining the color intensity is implemented by ascertaining the diversity of the image channel values from the individual image channels.

10. Method according to any of claims 7 to 9, **characterized in that** step bb) has the following steps:

- determining the probability of each pixel (410-419; 420-424) in a region of conspicuous color belonging to a particular class of anomalies (402),
- selecting and assigning the class having the highest probability to the respective pixel (410-419; 420-424), and
- selecting the most frequently occurring class among the total number of all pixels (410-419; 420-424) in the region of conspicuous color and assigning this class to the region of conspicuous color.

11. Computer-readable non-volatile medium having stored thereon a program which has program code parts which, when executed on a computer, result in the computer performing the steps of the method according to any of claims 7 to 10.

**Revendications**

1. Dispositif de détection d'anomalie sur des instruments médicaux, comportant une installation de traitement de données (14, 104, 154, 212, 304) et une unité de détection d'instruments (12, 102, 152, 302, 351, 371), dans lequel l'installation de traitement de données (14, 104, 154, 212, 304) comporte une unité d'affichage (38, 120, 170, 218), une base de données (28, 184, 236), une première interface (24, 180, 226) et une unité d'évaluation (26, 182, 232) et l'unité de détection d'instruments (12, 102, 152, 302, 351, 371) comporte un support (16, 108, 158, 312) et au moins une caméra (18, 110, 160, 252, 308, 310), dans lequel l'au moins une caméra (18, 110, 160, 252, 308, 310) est disposée et orientée de manière à ce qu'elle puisse acquérir des données d'images d'instruments médicaux (58, 64, 328, 400) disposés sur le support (16, 108, 158, 312) depuis au moins une perspective, et l'installation de traitement de données (14, 104, 154, 212, 304) est configurée de manière à ce qu'elle obtienne par l'intermédiaire de la première interface (24, 180, 226) des données d'images de l'au moins une caméra (18, 110, 160, 252, 308) et à ce qu'elle puisse stocker les données d'images obtenues dans la base de données (28, 184, 236) et qu'elle puisse examiner au moyen de l'unité d'évaluation (28, 182, 232) des régions qui présentent des anomalies (402), **caractérisé en ce que** l'installation de traitement de données (14, 104, 154, 212, 304) est en outre configurée de manière à ce qu'elle puisse classer les régions et à ce qu'elle puisse déterminer sur la base de ladite classification si les régions sont respectivement une partie normale de l'instrument (58, 64, 328, 400) ou une anomalie (302) et est configurée pour mettre en oeuvre les étapes consistant à :

aa) repérer des régions ressortant de manière colorée,
bb) classer les régions ressortant de manière colorée,
cc) déterminer sur la base de ladite classification si les régions ressortant de manière colorée sont une partie normale de l'instrument (58, 64, 328, 400) ou une anomalie (402), et
dd) déterminer le type de l'anomalie (402) de la région ressortant de manière colorée.

2. Dispositif selon la revendication 1, **caractérisé en ce que** les anomalies (402) sont des dommages ou des impuretés.

3. Dispositif selon la revendication 1 ou 2, **caractérisé en ce que** le support (16, 108, 158, 412) comporte un arrière-plan renforçant le contraste (66, 322, 352, 354, 372, 374).

4. Dispositif selon l'une quelconque des revendications 1 à 3, **caractérisé en ce que** la caméra (18, 110, 160, 252, 308, 310) est disposée de manière à ce que sa position puisse être modifiée afin qu'elle puisse détecter des données d'images depuis au moins deux perspectives.

5. Dispositif selon la revendication 3 ou 4, **caractérisé en ce que** le support (16, 108, 158, 312) comporte une surface de base transparente (320) et **en ce que** l'arrière-plan renforçant le contraste (62, 322) est disposé de manière à ce que sa position puisse être modifiée entre les deux côtés de la surface de base transparente (320).

6. Dispositif selon la revendication 3 ou 4, **caractérisé en ce que** le support (16, 108, 158, 312) comporte une surface de base transparente (320) et **en ce que** le dispositif comporte au moins deux arrière-plans renforçant le contraste (352, 354, 362, 374) qui sont respectivement disposés sur les côtés opposés de la surface de base transparente (320) de manière à ce qu'au moins un arrière-plan renforçant le contraste (352, 354, 372, 374) soit respectivement situé à l'arrière de l'instrument à détecter (58, 64, 328) depuis une perspective de caméra respective.

7.  Procédé destiné à détecter des anomalies sur des instruments médicaux, comportant un dispositif selon l'une quelconque des revendications 1 à 6, comprenant les étapes consistant à :

    a) monter au moins un instrument (58, 64, 328, 400) sur le support de l'unité de détection d'instruments (12, 102, 152, 302, 351, 371),
    b) acquérir des données d'images au moyen de l'au moins une caméra (18, 110, 160, 252, 308, 310),
    c) transmettre les données d'images à l'installation de traitement de données (14, 104, 154,212,304),
    d) analyser les données d'images pour détecter des anomalies (402) sur l'instrument (58, 64, 328, 400), comprenant les étapes consistant à :

       aa) repérer des régions ressortant de manière colorée,
       bb) classer les régions ressortant de manière colorée,
       cc) déterminer sur la base de ladite classification si les régions ressortant de manière colorée sont une partie normale de l'instrument (58, 64, 328, 400) ou une anomalie (402), et
       dd) déterminer le type de l'anomalie (402) de la région ressortant de manière colorée,

    e) communiquer les informations concernant les anomalies (402) à un utilisateur.

8.  Procédé selon la revendication 7, **caractérisé en ce que** l'étape aa) comporte les étapes consistant à :

    - déterminer l'intensité de coloration de chacun des pixels (410-419, 420-424),
    - comparer l'intensité de coloration à une valeur de seuil prédéterminée,
    - classer les pixels (410-419, 420-424) comme ressortant de manière colorée lorsque ladite valeur de seuil est dépassée, et
    - combiner tous les pixels ressortant de manière colorée voisins (416-419 ; 420-424) en une région ressortant de manière colorée.

9.  Procédé selon la revendication 8, **caractérisé en ce que** la détermination de l'intensité de coloration est réalisée par détermination de la différence entre les valeurs de canaux d'images des canaux d'images individuels.

10. Procédé selon l'une quelconque des revendications 7 à 9, **caractérisé en ce que** l'étape bb) comporte les étapes consistant à :

    - déterminer la probabilité que chaque pixel (410-419 ; 420-424) d'une région ressortant de manière colorée appartienne à une classe déterminée d'anomalies (402),
    - sélectionner et affecter la classe ayant la probabilité la plus grande au pixel respectif (410-419 ; 420-424), et
    - sélectionner la classe apparaissant de la manière la plus fréquente pour l'ensemble de tous les pixels (410-419 ; 420-424) de la région ressortant de manière colorée et affecter ladite classe à la région ressortant de manière colorée.

11. Support non volatil lisible par ordinateur sur lequel est stocké un programme d'ordinateur qui comporte des parties de codes de programmes pouvant être exécutées sur un ordinateur de manière à ce que ledit ordinateur mette en oeuvre les étapes du procédé selon l'une quelconque des revendications 7 à 10.

Fig.1

Fig.2

Fig.3

Fig.4

154

182

180

166    178    176

186

188

192

196

194

184

190

Fig.5

200

204

202

214

212

206

222

216

218

223    220

224    210    208

Fig.6

Fig.7

Fig.8

Fig.9

Fig.10

Fig.11

Fig.12

A          B                    406

404

402

400

Fig.13

410  411    412    413    414    415

404

419

418

417

402

416

A

406

Fig.14

Fig.15

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7997847 B2
- DE 202011050001 U1